# EUROPEAN PATENT APPLICATION

(11) **EP 3 848 019 A1**
(43) Date of publication of application: **14.07.2021**
(21) Application number: 20150813.2
(22) Date of filing: 08.01.2020
(51) Int. Cl.: A61K 8/60, A61K 8/73, A61K 8/97, A61K 8/9717, A61K 8/9789, A61Q 5/02, A61Q 5/12, A61Q 17/00, A61Q 19/00

(54) **COSMETIC COMPOSION COMPRISING A SAPONIN AND A PROBIOTIC COMPONENT**

(71) Applicant: CreaSearch BV, 3300 Tienen (BE)
(72) Inventor: BOOTEN, Karl, B-3300 Tienen (BE)
(74) Representative: IPLodge bv

(57) **Abstract**

The invention pertains to a cosmetic composition comprising: at least one saponin; and at least one prebiotic component selected from the group consisting of fructooligosaccharides, fructans, galactooligosaccharides, galactans, glucooligosaccharides, glucans, pectin, xylooligosaccharides, xylans, and mixtures thereof. The invention also pertains to a process for producing such a cosmetic composition and to a use of said cosmetic composition.

## Description

### Field of the Invention

The present invention relates to cosmetic compositions and to the use of such compositions in the preparation of a cosmetic product.

### Background

International patent application publication no. WO 93/00067 A1, in the name of BIOEUROPE, discloses cosmetic compositions providing a suitable medium for the development of beneficial endogenous flora, and including at least one oligosaccharide selected from the group consisting of gluco-oligosaccharides, fructo-oligosaccharides, α- and β-galacto-oligosaccharides and mixtures thereof. Examples of compositions are liquid soap, shampoo, body lotion, face cream and vaginal gel. That application does not disclose compositions comprising a saponin.

European patent application publication no. EP 1 380 284 A1, in the name of L'ORÉAL, discloses a detergent and conditioning composition comprising one or more anionic surfactants (a), one or more amphoteric, cationic and/or non-ionic surfactants (b) with a ratio by weight (a)/(b) of 1 or more and a polysaccharide which is a non-ionic or anionic fructan or starch hydrolysate with a dextrose equivalent below 20.

It is an object of embodiments of the present invention, to provide cosmetic compositions that have less impact on the diversity of the bacterial flora of the skin.

### Summary of the Invention

According to an aspect of the present invention, there is provided a cosmetic composition comprising: at least one saponin; and at least one prebiotic component selected from the group consisting of fructooligosaccharides, fructans, galactooligosaccharides, galactans, glucooligosaccharides, glucans, pectin, xylooligosaccharides, xylans, and mixtures thereof.

The term "oligosaccharide" is used herein to denote saccharide polymers containing a small number (up to ten) of monosaccharides (simple sugars). In particular, fructooligosaccharides (FOS) are short chains of fructose units, galactooligosaccharides (GOS) are short chains of lactose units, glucooligosaccharides are short chains of glucose units, and xylooloigosaccharides (XOS) are short chains of xylose units.

The term "polysaccharide" is used herein to denote saccharide polymers containing a larger number (more than ten) of monosaccharides (simple sugars). In particular, fructans are long chains of fructose units, galactans are long chains of lactose units, glucans are long chains of glucose units, and xylans are long chains of xylose units. The term fructan will be understood to include inulin, which are composed mainly of fructose units, and typically have a terminal glucose. Glucans include alpha-glucans (such as starch) and beta-glucans; in the present application, the term glucan will also be understood to include glucomannan and galactoglucomannan.

The term "saccharide" is used herein to denote monosaccharides, disaccharides, oligosaccharides, and polysaccharides. In the present application, the term "saccharide" will most often be used in statements that apply both to oligosaccharides and polysaccharides of a particular basic sugar.

Known cosmetic compositions tend to disturb the skin flora, an undesired effect which is believed to be due at least in part to the presence of surfactants. It is an advantage of the cosmetic composition according to the present invention that it reduces the disturbance of the skin flora. This means that it has a smaller impact on the diversity of the bacterial flora of the skin (either in the sense of increasing the diversity or decreasing the diversity), as assessed by comparing the Shannon diversity index of untreated skin with the Shannon index of skin treated with the composition. This unexpected effect is attributed to the combination of a specific surfactant (the at least one saponin) with a prebiotic component. According the invention, the prebiotic component being is in particular a fructooligosaccharide, a fructan, a galactooligosaccharides, a galactan, a glucooligosaccharide, a glucan, pectin, a xylooligosaccharide, a xylan, or a combination of any two or more of the aforementioned substances.

The combination of the at least one saponin and the at least one prebiotic component as described herein allows for the partial replacement of ingredients otherwise present in typical cosmetic products, which may be of synthetic or non-natural origin or which may be perceived less favorably by the general public. More in particular, the composition allows for the partial replacement of surfactants, including anionic surfactants.

In an embodiment, the cosmetic composition according to the present invention comprises an inulin-type polymer. The use of inulin and oligofructose has yielded very good results in the context of the present invention.

In an embodiment of the cosmetic composition according to the present invention, the at least one prebiotic component is present in the composition in a concentration from at least 0.01 wt% to at most 8 wt%, by weight relative to the total weight of the composition.

In an embodiment of the cosmetic composition according to the present invention, the at least one saponin is present in the composition in a concentration from at least 0.001 wt% to at most 15 wt%, by weight relative to the total weight of the composition.

In an embodiment, the cosmetic composition according to the present invention further comprises at least one of the following: an anionic surfactant, a cationic surfactant, an amphoteric surfactant, and a non-ionic surfactant, wherein said non-ionic surfactant is exclusive of a saponin.

In an embodiment, the cosmetic composition according to the present invention further comprises at least one oil selected from jojoba oil, isoamyl laurate, and cyclopentasiloxane; said at least one oil being comprised in a hydrophobic phase of a stable emulsion.

The term "emulsion" in the context of the present invention refers to a system whereby at least two non-miscible liquids are mixed together wherein one phase, the dispersed phase, gets dispersed in the other phase, the continuous phase. This typically involves mixing a phase component of hydrophobic nature, forming the hydrophobic phase in the envisaged emulsion, with a second phase component of hydrophilic nature, forming the hydrophilic phase in the envisaged emulsion. The "stability" of the emulsion refers to the ability of an emulsion to resist change in its properties over time, as will be explained in more detail hereinbelow.

It is an advantage of this embodiment that it allows the composition to remain in a stable emulsion form. This type of composition is particularly suitable for use as a leave-on product (e.g., a skin cream or lotion) or very specific types of rinse-off products (e.g. hair conditioner).

In an embodiment, the composition according to the present invention further comprises at least one emulsion breaking agent selected from the following:
- an antimicrobial agent, such as a medium-chain length linear vicinal diol, glyceryl caprylate, or caprylyl glycol;
- an electrolyte, such as MgSO₄ or NaCl;
- a cationic molecule, such as cationic surfactants such as cetrimonium chloride;
- an oxidizing agent, such as hydrogen peroxide in a concentration between 10 and 30% (v/v);
- a sunless tanning agent, such as dihydroxy acetone (DHA);
- a reducing agent, such as potassium thioglycolate;
- an alpha hydroxy acid, such as glycolic acid;
- an alpha and beta hydroxy acid, such as salicylic acid;
- a surfactant, such as cocamidopropyl betaine, sodium cocoyl glutamate, or sodium lauroyl glutamate;
- a C₁-C₇ alcohol, such as ethanol.

The inventor has found that the combination of the at least one saponin and the at least one prebiotic component according to the invention may result in obtaining a stable emulsion, even when adding amounts of emulsion breakers. Accordingly, a wide variety of emulsion-based products, in particular leave-on products, can be produced on the basis of the cosmetic composition of the present invention.

In an embodiment, the cosmetic composition according to the present invention is a leave-on cosmetic product or is part of a leave-on cosmetic product, and said at least one prebiotic component is present in the cosmetic product in a concentration of at least 0.01 wt% to at most 8 wt%, by weight relative to the total weight of the cosmetic product.

In a particular embodiment, the at least one saponin is present in the cosmetic product in a concentration of at least 0.001 wt% to at most 5 wt% by weight relative to the total weight of the cosmetic product.

The inventor has found that a concentration of saponin in this range is appropriate to obtain the desired effect in a leave-on product. In leave-on products, the main function of the saponin is to act as an emulsifier (or a co-emulsifier, as the case may be).

In a particular embodiment, the weight ratio of said at least one saponin to the total of all surfactants in the composition, excluding said at least one saponin, is at least 1:100 and at most 100:1.

In an embodiment, the cosmetic composition according to the present invention is a rinse-off cosmetic product or is part of a rinse-off cosmetic product, and said at least one prebiotic component is present in the cosmetic product in a proportion of at least 0.01 wt% to at most 8 wt%, by weight relative to the total weight of the cosmetic product

In a particular embodiment, the at least one saponin is present in the cosmetic product in a concentration of at least 0.1 wt% to at most 15 wt% by weight relative to the total weight of the cosmetic product.

The inventor has found that a concentration of saponin in this range is appropriate to obtain the desired effect in a rinse-off product. It shall be noted that the lower end of this range is higher than the lower end of the range presented above for leave-on products, and that the higher end of this range is likewise higher than the higher end of the range presented above for leave-on products. These higher concentrations are justified in view of the fact that rinse-off products will be in contact with the skin for much shorter durations than leave-on products. In rinse-off products, the saponin will contribute to the detergent or cleansing function of the product, along with the other surfactants present in the product.

In a particular embodiment, the weight ratio of said at least one saponin to the total of all surfactants in the composition, excluding said at least one saponin, is at least 1:100 and at most 100:1.

According to an aspect of the present invention, there is provided a process for preparing a cosmetic composition according to any of the preceding claims, the process comprising: extracting at least one saponin from its source using a mixture of water and a water-soluble alcohol; and combining said at least one saponin with at least one prebiotic component selected from the group consisting of fructooligosaccharides, fructans, galactooligosaccharides, galactans, gluco-oligosaccharides, glucans, pectin, xylooligosaccharides, xylans, and mixtures thereof.

This aspect of the invention is based on the insight of the inventor that the required saponin can efficiently be extracted from its plant source by use of a mixture of water and an alcohol. This extraction method specifically yields a saponin with improved color properties, which is important for the production of a visually pleasing cosmetic product.

In an embodiment of the process according to the present invention, the extraction is performed by use of a mixture of ethanol and water, wherein the ethanol: water ratio is between 60:40 and 80:20.

According to an aspect of the present invention, there is provided a use of the cosmetic composition as described above in the preparation of a rinse-off cosmetic product or a leave-on cosmetic product.

According to an aspect of the present invention, there is provided a medicinal composition for the treatment of acne vulgaris, the medical composition comprising: at least one saponin; and at least one prebiotic component selected from the group consisting of fructo-oligosaccharides, fructans, galactooligosaccharides, galactans, glucooligosaccharides, glucans, pectin, xylooligosaccharides, xylans, and mixtures thereof.

The inventor has surprisingly found that while a typical rinse-off composition applied to the skin results in an increase of abundance of *Cutibacterium acnes,* the bacterium that causes *acne vulgaris,* the abundance of this bacterium decreases when the composition is modified by replacing part of the anionic surfactant by the same amount of a saponin together with addition of inulin. This demonstrates that the saponin and the prebiotic component synergistically cooperate to maintain or restore a healthy bacterial skin flora.

### Detailed Description of Embodiments

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification do not necessarily refer to the same embodiment, although this may be the case. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

An aspect according to the invention relates to a cosmetic composition comprising at least one prebiotic component selected from the group consisting of fructooligosaccharides, fructans, galactooligosaccharides, galactans, glucooligosaccharides, glucans, pectin, xylooligosaccharides, xylans, and mixtures thereof; and at least one saponin.

According to embodiments of the invention, the at least one prebiotic component is present in the composition in a proportion which is at least 0.01 wt%, preferably at least 0.05 wt%, more preferably at least 0.1% by weight, even more preferably at least 0.2 wt%, more preferably at least 0.3 wt%, even more preferably at least 0.4 wt% and most preferably at least 0.5 wt%, relative to the total weight of the composition. It is further understood that the at least one prebiotic component is present in the composition in a proportion which is at most 8 wt%, preferably at most 7 wt%, more preferably at most 6 wt%, even more preferably at most 5 wt%, more preferably at most 4 wt%, even more preferably at most 3 wt%, more preferably at most 2 wt% and most preferably at most 1.5 wt%, by weight relative to the total weight of the composition.

According to embodiments of the invention, said cosmetic composition may be a cosmetic product, may be part of a cosmetic product or may be used in a cosmetic product. More in particular, said cosmetic composition is configured to be applied on parts of the human body, including the skin, hair, and mucous membranes.

A cosmetic product can refer herein to a leave-on cosmetic product, as well as a rinse-off cosmetic product. For the purpose of the invention, the term "rinse-off" refers herein to a cosmetic product which is used to be applied to the skin, the hair or mucous membranes, for example to clean them, and which is furthermore intended to be removed after application thereon. For the purpose of the invention, the term "leave-on" refers herein to a cosmetic product which is intended to stay in prolonged contact with the skin, the hair or mucous membranes. According to an aspect of the invention, there is provided a cosmetic product comprising the cosmetic composition described above. Said cosmetic product may be a rinse-off cosmetic product or a leave-on cosmetic product. It will be appreciated by the skilled in the art that the concentration for said at least one prebiotic component which will be typically applied in the final product is dependent on the type of cosmetic product.

When used as a leave-on product, the cosmetic compositions as described herein will typically form an emulsion, whereby the saponin acts as an emulsifier or a co-emulsifier. It was found that the combination of the at least one saponin and the at least one prebiotic component according to the invention may result in obtaining a stable emulsion, even when adding amounts further components that tend to act as emulsion breakers, as described herein.

When used as a rinse-off product, the cosmetic compositions as described herein will typically form a gel or a solution, although emulsions may be used for some specific applications. The hair and skin of mammals are prevented from becoming overly dry by an oily or waxy matter, called sebum, that is secreted by sebaceous glands. Over time, the accumulating sebum attracts dirt; the primary purpose of shampoo or soap is to remove the dirty sebum from the hair and/or skin. This is effected by surfactants present in the shampoo or soap. It is known in the state of the art to provide cosmetic compositions with several surfactants. As rinse-off compositions need to clean the skin and/or hair in a short period of time before they are washed off, such compositions typically contain at least one of anionic surfactants, amphoteric surfactants, cationic surfactants and non-ionic surfactants. Such surfactants typically allow for an acceptable cleansing effect which needs to be achieved during the relatively short period of time of a bath or a shower. For typical compositions, anionic surfactants make up the bulk of the surfactant ingredients. However, anionic surfactants also seem to have the highest effect on lipids and proteins which are naturally present on the skin and the hair, by e.g. denaturation of the latter. Anionic surfactants are therefore considered as a main source for damaging of such components.

When part of a rinse-off cosmetic product, the at least one prebiotic component is present in the product in a concentration which is at least 0.01 wt%, preferably at least 0.1 wt%, more preferably at least 0.5 wt% and most preferably at least 1 wt% with respect to the total weight of the cosmetic product. Furthermore, the at least one prebiotic component is present in the cosmetic product in a concentration which is at most 8 wt%, preferably at most 7 wt% and most preferably at most 6 wt% with respect to the total weight of the product.

When part of a leave-on cosmetic product, the at least one prebiotic component is present in the product in a concentration which is at least 0.01 wt%, preferably at least 0.1 wt%, more preferably at least 0.5 wt% and most preferably at least 1 wt% with respect to the total weight of the cosmetic product. Furthermore, the at least one prebiotic component is present in the cosmetic product in a concentration which is at most 8 wt%, preferably at most 7 wt%, more preferably at most 6 wt%, even more preferably at most 5 wt% and most preferably at most 4 wt%, with respect to the total weight of the product.

According to preferred embodiments of the invention, the at least one prebiotic component is or comprises a **fructose-based polymer** (oligosaccharide or polysaccharide), i.e. a polymer of fructose molecules, which may or may not bear one terminal glucose unit. Typically, fructose-based polymers are subdivided into levan and inulin.

According to embodiments of the invention, said fructose-based polymer is of the levan type. Levan-type polymers are composed of fructooligosaccharide of polyfructose molecules wherein the fructosyl units are mostly connected to each other by β-2,6-linkages. Levan is naturally synthesized by certain plant species and by certain bacteria.

According to embodiments of the invention, said fructose-based polymer is of the inulin type. Inulin-type polymers are composed of fructooligosaccharide or polyfructose molecules of which the fructosyl units are exclusively or mainly connected to each other by β-2,1-linkages and which furthermore may or may not bear one terminal glucose unit. The molecules can be linear, namely when all the fructosyl units are exclusively connected to each other by β-2,1- linkages, but can also be branched, namely when the fructosyl units are connected to a certain extent but for less than 50 percent to each other by β-2,6-linkages. The degree of polymerization (DP) is expressed on the basis of the number of fructosyl units forming the polymer. Inulin-type polymers composed of molecules with a DP ranging from 2 to about 100,000 are commonly named "inulin". Inulin-type polymers composed of molecules with a DP ranging from 2 to 10 are commonly and interchangeably named "oligofructose", "fructo-oligosaccharide" or "inulo-oligosaccharide". The term "inulin-type polymer", as used herein, refers both to inulin and oligofructose. For the sake of completeness, it is noted that the term "inulin-type fructan" is sometimes used in literature to refer to the notion of "inulin-type polymer" as defined above.

Inulin-type polymer is naturally synthesized by many plant species but can also originate from bacterial activity. Inulin-type polymer naturally occurs as a polydisperse mixture of linear and/or branched polyfructose molecules that is characterized *inter alia* by the number-average degree of polymerization (avDP) of the polyfructose molecules. The DP and the avDP of inulin-type fructan are dependent on the origin of the fructan Typically, inulin from plant origin has a DP ranging from 2 to about 200, mostly from 2 to about 100.

Inulin is mostly obtained by isolation from roots of chicory (*Cichorium intybus*), from tubers of Jerusalem artichoke (*Helianthus tuberosus*) and Dahlia, and from Agave, preferably from the head of the Blue Agave. In the native form, namely without a treatment to increase or reduce the DP, chicory inulin has a DP ranging from 2 to about 70 and an average degree of polymerization of about 10 to about 12. Similarly, Jerusalem artichoke inulin has a DP ranging from 2 to about 40 and an average degree of polymerization of about 6; dahlia inulin an average degree of polymerization of about 15 to about 20; and agave inulin an average degree of polymerization of about 14 to about 18. Agave inulin is typically more branched than inulin isolated from chicory.

Oligofructose can be obtained at industrial scale by partial (acidic or enzymatic) hydrolysis of inulin from plant origin or from bacterial origin, or by enzymatic synthesis from saccharose according to well-known techniques (e.g., Anwar et al. Appl Environ Microbiol. 2008 Jun; 74(11): 3426-3433.). Typically, such oligofructose then has a DP ranging from 2 to about 9.

At commercial scale, inulin is mainly manufactured from chicory roots. Chicory inulin is for example available as a spray-dried powder, and can be obtained in various grades, characterized inter alia by a varying DP and average degree of polymerization.

According to preferred embodiments of the invention, the inulin-type polymer is **oligofructose.** Preferably, said oligofructose is an oligofructose with a DP which is at least 2, more preferably at least 3. It is further understood that said oligofructose has a DP which is at most 10, preferably at most 9 and more preferably at most 8.

According to alternative embodiments of the invention, the inulin-type polymer is **inulin** with a DP ranging from 11 to about 200, more preferably inulin with a DP ranging from 11 to about 100.

According to preferred embodiments of the invention, said inulin-type polymer is an inulin or oligofructose from plant origin; more preferably, the inulin-type polymer is chicory inulin. Alternatively, said inulin-type polymer is inulin from Jerusalem artichoke, inulin from Dahlia, or said inulin-type polymer is a branched inulin, for example agave inulin.

In still other alternative embodiments of the invention, the inulin-type polymer is obtained by partial (acidic or enzymatic) hydrolysis of inulin from plant origin or from bacterial origin, or by enzymatic synthesis from saccharose.

According to embodiments of the invention, the at least one prebiotic component is or comprises a **galactooligosaccharide.** Galactooligosaccharides (GOSs) are oligosaccharides composed of different galactosyl residues (from 2 to 9 units) and a terminal glucose linked by β-glycosidic bonds, such as β-(1-2), β-(1-3), β-(1-4), and β-(1-6). They naturally occur at low concentrations in the milk of many animals, including humans and cows, but they also can be produced by chemical glycosylation or biocatalysis

According to embodiments of the invention, the at least one prebiotic component is or comprises a **glucooligosaccharide.** glucooligosaccharides are formed according to the general formula (0-α-D-glucopyranosyl)n-A, where A is the residue of a glucose-accepting sugar (such as maltose, isomaltose, isomaltotriose, α-methylglucoside, and glucose). The coefficient *n* = may be in the range from 1 to 10. The glucosidic bonds are of the α(1→6) type and optionally α(1→2) and/or α(1→3). The α(1→2) bond, if present, is situated at the non-reducing end or constitutes a branching point. Processes for the synthesis of such glucooligosaccharides are known in the art, and can be found *inter alia* in European patent application publication no EP 0 325 872 A1 and US patent no. 2,726,190.

According to embodiments of the invention, the at least one prebiotic component is or comprises a **glucan,** preferably a beta-glucan. Beta-glucans comprise a group of β-D-glucose polysaccharides naturally occurring in the cell walls of cereals, bacteria, and fungi, with significantly differing physicochemical properties dependent on source. Typically, beta-glucans form a linear backbone with 1-3 β-glycosidic bonds. Beta-glucans vary with respect to molecular mass, solubility, viscosity, branching structure, and gelation properties. By convention not all β-D-glucose polysaccharides are categorized as beta-glucans; e.g. cellulose is not conventionally considered a beta-glucan, as it is insoluble and does not exhibit the same physicochemical properties as other cereal or yeast beta-glucans, so it is not considered to be comprised in the term "beta-glucan" as used herein.

According to embodiments of the invention, the at least one prebiotic component is or comprises **resistant starch.** Resistant starch (RS) his used herein to denote the sum of starch and products of starch degradation not absorbed in the small intestine of healthy individuals. Starch is a mixture of two polymers: amylose and amylopectin. Natural starches consist of about 10%-30% amylase and 70%-90% amylopectin. Amylose is a linear polysaccharide composed entirely of D-glucose units joined by the α-1,4-glycosidic linkages. Amylopectin is a branched-chain polysaccharide composed of glucose units linked primarily by α-1,4-glycosidic bonds but with occasional α-1,6-glycosidic bonds, which are responsible for the branching. A molecule of amylopectin may contain many thousands of glucose units with branch points occurring about every 25-30 units.

According to embodiments of the invention, the at least one prebiotic component is or comprises **glucomannan.** Glucomannan is mainly a straight-chain polymer, with a small amount of branching. The component sugars are β-(1→4)-linked D-mannose and D-glucose in a ratio of 1.6:1. The degree of branching is about 8% through β-(1→6)-glucosyl linkages. Glucomannan is a water-soluble and appears as a hemicellulose component in the cell walls of some plant species.

According to embodiments of the invention, the at least one prebiotic component is or comprises **pectin.** Pectin is a structural acidic heteropolysaccharide contained in the primary cell walls of terrestrial plants. Its main component is galacturonic acid, a sugar acid derived from galactose. Several distinct polysaccharides have been identified and characterized within the pectic group. Heterogalacturonans are linear chains of α-(1-4)-linked D-galacturonic acid. Substituted galacturonans are characterized by the presence of saccharide appendant residues (such as D-xylose or D-apiose in the respective cases of xylogalacturonan and apiogalacturonan) branching from a backbone of D-galacturonic acid residues. Rhamnogalacturonan I pectins (RG-I) contain a backbone of the repeating disaccharide: 4)-α-D-galacturonic acid-(1,2)-α-L-rhamnose-(1. From many of the rhamnose residues, sidechains of various neutral sugars branch off. The neutral sugars are mainly D-galactose, L-arabinose and D-xylose, with the types and proportions of neutral sugars varying with the origin of pectin.

According to embodiments of the invention, the at least one prebiotic component is or comprises a **xylan,** preferably an arabinoxylan. Arabinoxylans consist of a linear backbone chain of β-D-xylopyranosyl residues linked through (1,4) glycosidic linkages. An α-L-arabinofuranosyl residue is attached to some of the D-xylopyranosyl residues at O-2, O-3, and/or at both O-2,3 positions. The majority of L-arabinofuranosyl residues are present as monomeric substituents, while a small portion are oligomeric side chains consisting of two or more L- arabinofuranosyl residues linked via 1,2, 1,3 and 1,5 linkages. Arabinoxylans have been found in all major cereal grains, including rye, wheat, barley, oats, sorghum, maize, millet, psyllium, flaxseed, pangola grass, bamboo shoot and rye grass.

**Saponins** are a class of chemical compounds which can be structurally defined in that they consist of an aglycone unit (or "sapogenin unit"), derived from a lipophilic triterpene or sterol unit, linked to one or more hydrophilic glycoside moieties. The sapogenin unit's carbon skeleton may be saturated or unsaturated and/or comprise a heteroatom such as nitrogen. The glycoside moiety contains sugars such as galactose, glucose, glucuronic acid, methyl pentose, rhamnose, and xylose. Due to the hydrophilic nature of the glycoside moieties, as well as the lipophilic nature of the triterpene or sterol unit, saponins possess surface-active properties. Saponins are further known to have to a greater or lesser extent an antimicrobial, antiherbivore and/or cytotoxic activity. Their role in nature is likely to be in the defense against pathogens, pests and predators.

Saponins may be obtained from natural sources. For the purpose of the invention, the term "natural" as in "natural component" refers to the situation wherein such a component contained in the composition is obtained from a natural source (plant source or non-plant source, e.g. animal source or marine organisms) in a substantially unmodified form, namely in the form which exists in the material of natural origin. Referring specifically to the category of marine organisms, it is known that saponins may be obtained *inter alia* from sea cucumbers and starfish.

Saponins are considered as non-ionic surfactants although they can have a negative charge due to the presence of one or more carboxylic groups.

An aspect of the invention relates to a process for preparing a cosmetic composition as described above, the process comprising: extracting at least one saponin from its source using a mixture of water and a water-soluble alcohol; and combining said at least one saponin with at least one prebiotic component selected from the group consisting of fructooligosaccharides, fructans, galactooligosaccharides, galactans, glucooligosaccharides, glucans, pectin, xylooligosaccharides, xylans, and mixtures thereof.

According to preferred embodiments of the invention, the saponins used herein are obtained from a plant source or a mixture of different sources, such as multiple plant sources.

Said plant source may be tea (including *Camellia sinensis and Camelia oleifeira*), species from the *Sapindus* Genus (including *Sapindus delavayi, Sapindus oahuensis, Sapindus mukorossi, Sapindus marginatus, Sapindus saponaria, Sapindus trifoliatus, Saponaria officinalis*), *Quillaja saponaria,* species of the Genus *Yucca* (including *Yucca Schidigera*), species of the Genus *Gynostemma,* shikakai, soybeans, beans, mung beans, peas (*Pisum sativum*), alfalfa, spinach, sugar beet, quinoa, liquorice, sunflower, horse chestnut, species from the Genus *Panax* (including *Panax quinquefolius, Panax japonicus*), oats, capsicum peppers, eggplant (aubergine), tomato seed, species of the *Allium* Genus, asparagus, yam, fenugreek, *Bupleurum falcatum, Desmodium adscendens, Gypsophila,* and *Styrax japonica.*

For the purpose of extracting the saponin material, any part of the plant may be used, including leaves, stems, roots, bulbs, blossom and fruit (including the skin, flesh and seed of the fruit).

According to preferred embodiments, said plant source is tea (preferably *Camelia sinensis* or *Camellia oleifera*), *Quillaja Saponaria* or a species of the *Sapindus* Genus, and said at least one saponin comprises a tea saponin or saponin from *Quillaja Saponaria* or at least one species of the *Sapindus* Genus, or mixtures thereof.

According to embodiments, said at least one saponin is a tea saponin or a mixture of saponins from *Camelia sinensis* or *Camellia oleifera.*

According to embodiments, said at least one saponin is a saponin from at least one species of the *Sapindus* Genus, or mixtures thereof.

According to embodiments, said at least one saponin is a saponin from *Quillaja Saponaria.*

According to embodiments of the invention, the at least one saponin is present in the composition in a proportion which is at least 0.001 wt%, preferably at least 0.005 wt%, more preferably at least 0.01% by weight, even more preferably at least 0.05 wt%, more preferably at least 0.1 wt%, even more preferably at least 0.2 wt% and most preferably at least 0.3 wt%, relative to the total weight of the composition. It is further understood that the at least one saponin is present in the composition in a proportion which is at most 15 wt%, preferably at most 12 wt%, more preferably at most 9 wt%, even more preferably at most 8 wt%, more preferably at most 7 wt%, even more preferably at most 6wt%, and most preferably at most 5 wt%, by weight relative to the total weight of the composition.

As mentioned here above, said cosmetic composition may be part of a cosmetic product. Said cosmetic product may be a rinse-off cosmetic product or a leave-on cosmetic product. It will be appreciated by the skilled in the art that the concentration for said at least one saponin which is typically applied in the final product will depend on the type of cosmetic product.

When part of a rinse-off cosmetic product, the at least one saponin is present in the product in a concentration which is at least 0. 01 wt%, preferably at least 0.1 wt%, more preferably at least 0.5 wt%, even more preferably at least 1 wt%, more preferably at least 2 wt%, even more preferably at least 3 wt%, more preferably at least 4wt%, and most preferably at least 5 wt% with respect to the total weight of the cosmetic product. Furthermore, the at least one saponin is present in the cosmetic product in a concentration which is at most 15 wt%, preferably at most 14 wt%, more preferably at most 13 wt%, even more preferably at most 12 wt%, more preferably at most 11 wt%, even more preferably at most 10 wt% and most preferably at most 9 wt% with respect to the total weight of the product.

When part of a leave-on cosmetic product, the at least one saponin is present in the product in a concentration which is at least 0. 001 wt%, preferably at least 0.005 wt%, more preferably at least 0.01 wt%, even more preferably at least 0.05wt%, more preferably at least 0.1 wt% and most preferably at least 0.2 wt% with respect to the total weight of the cosmetic product. Furthermore, the at least one saponin is preferably present in the cosmetic product in a concentration which is at most 5 wt%, more preferably at most 4.5 wt %, even more preferably at most 4 wt%, more preferably at most 3.5 wt%, even more preferably at most 3 wt%, more preferably at most 2.5 wt%, even more preferably at most 2 wt%, and most preferably at most 1.5 wt% with respect to the total weight of the product.

As rinse-off compositions typically have a surfactant concentration which may exceed 15 wt% with respect to the total weight of the product, it will be appreciated that the concentration of the at least one saponin will be relatively high when present in rinse-off compositions or products in comparison to leave-on products or compositions, where the at least one saponin will be used in more limited amounts in its capacity as an emulsifier. Moreover, in embodiments of the invention, it becomes possible to use the at least one saponin at lower concentrations than for a typical emulsifier while still resulting in a stable emulsion.

In a product having the form of an emulsion the hydrophobic phase typically comprises at least one oil and/or at least one other component. Said at least one other component can be selected from fatty substances, hydrophobic particles and/or waxes. For the purpose of the present invention, the term "oil" refers to a substance which is liquid at ambient temperature, and which is hydrophobic. Said oils can be mineral oils, vegetable oils or silicone oils.

In a preferred embodiment, the hydrophobic phase is an oily phase, comprising an oil, by preference selected from the group consisting of jojoba oil, isoamyl laurate, iso-hexadecane cyclopentasiloxane and mixtures thereof. Hence, according to embodiments of the cosmetic composition of the invention, said cosmetic composition forms an emulsion further comprising at least one of jojoba oil, isoamyl laurate and cyclopentasiloxane.

In the case an oily phase is dispersed in an aqueous phase, such emulsion is called an "oil-in-water emulsion" or "o/w emulsion". When the aqueous phase is dispersed in the oily phase, such emulsion is called a "water-in-oil emulsion" or "w/o emulsion".

According to embodiments of the invention, said cosmetic composition forms an o/w emulsion.

Multiple emulsions are a specific type of emulsions whereby two types can be considered, oil in water in oil (o/w/o) and water in oil in water (w/o/w).

In the case of an o/w/o emulsion, firstly an oil-in-water emulsion is prepared by using an appropriate emulsifier, comprising a saponin or an emulsifier with a high hydrophilic lipophilic balance (HLB) number. This o/w emulsion will then be emulsified in an oil phase with an appropriate w/o emulsifier with a low HLB number.

In case of a w/o/w emulsion, firstly a water-in-oil emulsion is prepared with w/o emulsifier having a low HLB number. This w/o emulsion will then be emulsified in water, together with an appropriate o/w emulsifier, comprising a saponin or an emulsifier with a high HLB number.

According to alternative embodiments of the invention, said cosmetic composition forms the o/w emulsion of an o/w/o emulsion or the o/w emulsion of an w/o/w emulsion.

According to preferred embodiments of the invention, the cosmetic composition forms a stable emulsion, referring to the ability of an emulsion to resist change in its properties over time. There are three types of instability in emulsions: irreversible flocculation, coalescence, and Ostwald ripening. Ostwald ripening is generally found in water-in-oil emulsions, while flocculation is found in oil-in-water emulsions.

Flocculation occurs when there is an attractive force between the droplets of the dispersed phase, said droplets forming flocs as a consequence, like bunches of grapes.

Coalescence occurs when droplets bump into each other and combine to form a larger droplet, so the average droplet size increases over time. In the case of solid phase emulsions, the term irreversible flocculation is used. This is applicable for example for emulsion polymers and pigments.

Ostwald ripening is the process of disappearance of small droplets by dissolution and deposition on the larger particles or droplets, as caused by the solubility differential between smaller and larger droplets.

Emulsions can also undergo creaming, whereby, in the particular case of o/w emulsions, the droplets of the dispersed phase rise to the top of the emulsion under the influence of buoyancy, or under the influence of the centripetal force induced when a centrifuge is used. It should be noted that creaming does not implicate coalescence and vice versa. Creaming, being a phase separation caused by oil droplets moving towards the top of the system, and/or sedimentation is not considered herein as an instability. Since the speed of creaming is influenced by the viscosity of the continuous phase and the droplet diameter, following the law of Stokes, large oil droplets in a macroemulsion will move faster to the top than the smaller oil droplets. It also means that if the viscosity of the continuous phase is high enough, creaming will not occur, even when the droplets are big. Increasing the viscosity of an emulsion can easily be obtained by the addition of thickening agents. In the particular case of a nanoemulsion, with oil droplets smaller than 500 nm, the phenomenon of creaming may disappear since the Brownian movement of the droplets become more important than the gravity forces.

On the other hand, nanoemulsions are more sensitive for Ostwald ripening than macroemulsions, since the speed of Ostwald ripening is in function of the droplet diameter, which is in the case of a nanoemulsion several times smaller than in the case of a macroemulsion.

An appropriate "surface active agent" (or "surfactant" or "tensioactive" or "emulsifier") can increase the kinetic stability of an emulsion so that the size of the droplets does not change significantly with time. It is then said to be stable. This means that an emulsion can be called stable when neither coalescence nor Ostwald ripening occurs in a certain period of time and at a given temperature.

For the purpose of the remainder of this description, an operational definition of emulsion stability is implied, whereby an emulsion is considered stable if it does not give rise to any of the above mentioned three types of instability after uninterrupted storage in an oven for at least 4 weeks at 50 °C.

Some compounds/molecules are known to be difficult to incorporate in emulsions since they cause coalescence. This coalescence can occur immediately after the emulsion has been prepared or in a period shorter than the desired stability requirements or at a temperature lower than the desired stability requirement. Such molecules/compounds will further be called "emulsion breakers". As examples can be given; electrolytes, cationic molecules including cationic surfactants, medium-chain length linear vicinal diols, reducing agents like potassium thioglycolate, oxidizing agents like hydrogen peroxide, sunless tanning agents like dihydroxy acetone (DHA).

The present invention does not exclude the presence of additional components in the cosmetic composition, other than the at least one saponin and the at least one prebiotic component. According to embodiments of the cosmetic composition according to the present invention, additional components comprise one or more compounds from the following classes: detergents and anti-bacterial agents; surfactants, more in particular anionic, non-ionic, amphoteric and/or cationic surfactants; cleansing agents, preservatives and pH-stabilizing agents, lather-forming agents, conditioning agents, thickening agents, coloring agents, perfuming agents, antioxidants, plant extracts, and combinations thereof. Such compounds are well known in the state of the art.

For the preparation of an emulsion with a certain stability, there is a need for the presence of a surface-active molecule that can lower the interfacial tension of the two non-miscible liquids together with the input of mixing energy that can be provided through shaking, stirring and/or homogenizing.

According to embodiments of the invention, said cosmetic composition further comprises at least one surfactant, other than saponin.

Surfactants are compounds that lower the surface tension (or interfacial tension) between two liquids or between a liquid and a solid. They are usually organic compounds that are amphiphilic, meaning they contain both hydrophobic groups (their tails) and hydrophilic groups (their heads). Therefore, a surfactant contains both a water-insoluble (or oil-soluble) component and a water-soluble component. Surfactants will diffuse in water and adsorb at interfaces between air and water or at the interface between oil and water, in the case where water is mixed with oil. The water-insoluble hydrophobic group may extend out of the bulk water phase, into the air or into the oil phase, while the water-soluble head group remains in the water phase. Commonly encountered surfactants typically belong to one of the following classes: anionic, cationic, amphoteric (zwitterionic) and non-ionic.

According to embodiments of the invention, said cosmetic composition further comprises at least one of a non-ionic surfactant other than saponin, an anionic surfactant, a cationic surfactant and an amphoteric surfactant.

According to embodiments of the invention, the cosmetic composition further comprises at least one non-ionic surfactant, which is selected from the following:
- fatty alcohols, such as cetyl alcohol, stearyl alcohol, and cetostearyl alcohol (consisting predominantly of cetyl and stearyl alcohols), and oleyl alcohol;
- non-ionic surfactants whereby the alcohol function has been substituted by a variety of different polar groups, such as polyethylene glycol alkyl ethers like octaethylene glycol monododecyl ether and pentaethylene glycol monododecyl ether, polypropylene glycol alkyl ethers, glucoside alkyl ethers like decyl glucoside, lauryl glucoside and octyl glucoside, sucrose alkyl esters, carbohydrate alkyl carbamates like inulin lauryl carbamate, glycerol alkyl esters like glyceryl laurate, glyceryl mono-stearate, polyoxyethylene glycol sorbitan alkyl esters like polysorbate, sorbitan alkyl esters like Spans, cocamide MEA, cocamide DEA, dodecyldimethylamine oxide, block copolymers of polyethylene glycol and polypropylene glycol like poloxamers and polyethoxylated tallow amine (POEA);
- ethoxylated fatty acids.

According to embodiments of the invention, the cosmetic composition further comprises at least one anionic surfactant, which is selected from the following:
- anionic surfactants containing anionic functional groups at their head, such as sulfate, sulfonate, sulfosuccinates, sarcosinates, glycine, glutamate phosphate, and carboxylates;
- alkyl sulfates including ammonium lauryl sulfate, sodium lauryl sulfate (sodium dodecyl sulfate, SLS, or SDS), and their related alkyl-ether sulfates, such as sodium laureth sulfate (sodium lauryl ether sulfate or SLES).

According to embodiments of the invention, the cosmetic composition further comprises at least one cationic surfactant, which is selected from the following:
- cationic surfactants with permanently charged quaternary ammonium salts including cetrimonium bromide (CTAB), cetrimonium chloride, cetylpyridinium chloride (CPC), behentrimonium chloride, stearyl dimonium hydroxypropyl laurylglucosides chloride

However, mostly these cationic surfactants are difficult to incorporate in emulsions and can be considered as emulsion breakers.

Zwitterionic (amphoteric) surfactants have both cationic and anionic centers attached to the same molecule. The cationic part is based on primary, secondary, or tertiary amines or quaternary ammonium cations. The anionic part can be more variable.

According to embodiments of the invention, the cosmetic composition further comprises at least one amphoteric surfactant, which is selected from the following:
- certain sulfonates, including the sultaines CHAPS (3-[(3-Cholamidopropyl)-dimethylammonio]-1-propanesulfonate) and cocamidopropyl hydroxysultaine;
- betaines such as cocamidopropyl betaine having a carboxylate with the ammonium;
- amphoteric surfactants having a phosphate anion with an amine or ammonium, such as the phospholipids phosphatidylserine, phosphatidylethanolamine, phosphatidylcholine, and sphingomyelins.

According to embodiments of the invention, saponins may be used as a surface-active agent only, as an emulsifier only, or as both; saponins may be used alone or in combination with other surfactants/emulsifiers/tensio-actives.

Preferably, the weight ratio of said at least one saponin and the total of all surfactants in the composition, excluding said at least one saponin, is at least 1:100, preferably at least 1:90, more preferably at least 1:80, even more preferably at least 1:70, more preferably at least 1:60, even more preferably at least 1:50, more preferably at least 1:40 and most preferably at least 1:30. It is further understood that said weight ratio is at most 100:1, preferably at most 70:1, more preferably at most 30:1, even more preferably at most 10:1, more preferably at most 1:1, even more preferably at most 1:2, more preferably at most 1:5 and most preferably at most 1:7.

According to embodiments of the invention, said cosmetic composition further comprises one or more emulsion breaking agents. Preferably, said one or more emulsion breaking agents are selected from:
- an antimicrobial agent such as a medium-chain length linear vicinal diols, glyceryl caprylate, pentylene glycol, or caprylyl glycol;
- an electrolyte such as MgSO₄, or NaCl;
- a cationic molecule such as cationic surfactants such as cetrimonium chloride;
- an oxidizing agent such as hydrogen peroxide up to concentration between 10 and 30%;
- a sunless tanning agent such as dihydroxy acetone (DHA);
- reducing agents such as potassium thioglycolate;
- alpha hydroxy acids such as glycolic acid;
- surfactants such as cocamidopropyl Betaine, sodium cocoyl glutamate, sodium lauroyl glutamate;
- C1-C7 alcohols, such as ethanol; and
- perfume compounds.

It was found that emulsions according to the invention remained stable even after addition of specific amounts of one of said emulsion breaking agents. This unexpected effect is further illustrated in Experiment 7, described below, for typical concentrations of emulsion breaking agents.

According to embodiments of the invention, the cosmetic composition forms an emulsion wherein the hydrophilic phase forms an aqueous phase, comprising water.

Preferably, the hydrophilic phase contains water and hydrophilic adjuvants, including monoalcohols containing 2 to 8 carbon atoms, for instance ethanol and isopropanol; and polyols, for instance glycerol; glycols, for instance pentylene glycol, propylene glycol, butylene glycol, isoprene glycol and polyethylene glycols such as PEG-8; sorbitol; sugars such as glucose, fructose, maltose, lactose or sucrose, and mixtures thereof.

However, lower alcohols, such as ethanol are also known and used as emulsion breaking agent.

The polyol that is miscible with water at room temperature (25 °C) may be chosen from polyols containing 2 to 20 carbon atoms, preferably containing 2 to 10 carbon atoms and more preferably containing 2 to 6 carbon atoms, such as glycerol, propylene glycol, butylene glycol, pentylene glycol, hexylene glycol, dipropylene glycol or diethylene glycol; glycol ethers (especially containing 3 to 16 carbon atoms), such as mono-, di- or tripropylene glycol (C1-C4) alkyl ethers or mono-, di- or triethylene glycol (C1-C4) alkyl ethers, and mixtures thereof.

According to embodiments of the invention, the cosmetic composition forms an emulsion wherein the hydrophobic phase comprises at least one oil and/or at least one other component.

Said at least one other component can be selected from fatty substances, hydrophobic particles and/or waxes.

Mention may be made especially of oils, fatty esters, waxes and butters, which may be, respectively, of natural (animal or plant) origin or synthetic origin.

Fatty substances of natural origin will preferentially be used, such as plant oils, fatty esters of plant origin and waxes or butters of plant origin.

Fatty substances can also be gums and pasty fatty substances, of animal, plant, mineral or synthetic origin, and mixtures thereof.

The oily phase may also comprise any common liposoluble or lipodispersible additive.

Preferably, the hydrophobic phase contains at least one cosmetic oil.

According to embodiments of the invention, said oil is a volatile oil.

The term "volatile oil" means any non-aqueous medium that is capable of evaporating from the skin or the lips in less than one hour, especially having a vapor pressure, at room temperature and atmospheric pressure, ranging from 10⁻³ to 300 mmHg (0.13 Pa to 40 000 Pa). As volatile oils that may be used in the invention, it is possible to use non-silicone volatile oils, especially C₈-C₁₆ isoparaffins, for instance isododecane, or isodecane.

According to embodiments of the invention, said oil is a non-volatile oil.

The term "non-volatile oil" means an oil that is capable of remaining on the skin at room temperature (25 °C) and atmospheric pressure for at least one hour and that especially has a non-zero vapor pressure at room temperature (25 °C) and atmospheric pressure, of less than 0.01 mmHg (1.33 Pa).

As non-volatile oils that may be used in the invention, mention may be made of non-silicone and especially hydrocarbon-based non-volatile oils, such as liquid paraffin (or petroleum jelly), squalane, hydrogenated polyisobutylene (parleam oil), perhydrosqualene, mink oil, soybean oil, sweet almond oil, beauty- leaf oil, palm oil, grapeseed oil, sesame seed oil, corn oil, arara oil, rapeseed oil, sunflower oil, cottonseed oil, apricot oil, castor oil, avocado oil, jojoba oil, olive oil, argan oil, virgin sweet almond oil, apricot kernel oil, rice bran oil, camellia oil or cereal germ oil; as preferred oils, jojoba oil or apricot kernel oil or any other triglyceride, and mixtures thereof, will be used; lanolic acid, oleic acid, lauric acid or stearic acid esters; esters derived from long-chain acids or alcohols (i.e. chains containing from 6 to 20 carbon atoms), such as isoamyl laurate, especially the esters of formula RCOOR' in which R represents a higher fatty acid residue containing from 7 to 19 carbon atoms and R' represents a hydrocarbon-based chain containing from 3 to 20 carbon atoms, in particular C12-C36 esters, such as isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, bis (2-ethylhexyl) succinate, diisostearyl malate, or glyceryl or diglyceryl triisostearate,- higher fatty acids, especially of C14- C22 such as myristic acid, palmitic acid, stearic acid, behenic acid, oleic acid, linoleic acid, linolenic acid or isostearic acid; higher fatty alcohols, especially of C16-C22 such as cetanol, oleyl alcohol, linoleyl alcohol, linolenyl alcohol, isostearyl alcohol or octyldodecanol; and mixtures thereof.

According to embodiments of the invention, said oil is a silicone oil.

Silicone oils, for instance volatile or non-volatile polymethylsiloxanes (PDMSs) containing a linear or cyclic silicone chain, which are liquid or pasty at room temperature, especially volatile silicone oils, in particular, cyclopentasiloxane, cyclopolydimethylsiloxanes (cyclomethicones) such as cyclohexadimethylsiloxane and cyclopentadimethylsiloxane; polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyltrimethylsiloxydiphenylsiloxanes, diphenyl dimethicones, diphenylmethyl-diphenyltrisiloxanes, 2-phenylethyltrimethylsiloxysilicates and polymethylphenylsiloxanes cross-polymers with vinyl groups and alkyl groups, such as vinyl dimethicone crosspolymer, C₃₀-C₄₅ alkyl cetearyl dimethicone crosspolymer or polymers such as polymethylsilsesquioxane, and mixtures thereof.

The choice of the oils is especially made as a function of the desired aim. Thus, the triglycerides and the plant oils such as apricot oil or olive oil are preferred for compositions intended for cosmetic application on dry skin, whereas fatty acid esters, which are lighter, are preferred for compositions intended for normal or combination skin.

Other fatty substances that may be present in the oily phase are, for example, fatty acids containing from 8 to 30 carbon atoms, for instance stearic acid, lauric acid or palmitic acid; pasty fatty substances, for instance petroleum jelly or lanolin.

Similarly, the oily phase may comprise a fat which is a derivative of a fatty acids such as described above and glycerol. Examples fats used in cosmetics comprise butters such as Sheabutter, Cupuacu butter and Mango butter.

Waxes are organic compounds that characteristically consist of long alkyl chains. They may also include various functional groups such as fatty acids, primary and secondary long chain alcohols, unsaturated bonds, aromatics, amides, ketones, and aldehydes. They frequently contain fatty acid esters as well. Synthetic waxes are often long-chain hydrocarbons (alkanes or paraffins) that lack functional groups.

Examples thereof are waxes, for instance beeswax, carnauba wax, candelilla wax, paraffin wax, lignite wax, microcrystalline waxes, ceresin, ozokerite, synthetic waxes such as polyethylene waxes, polymethylene waxes and Fischer-Tropsch waxes.

Said substances may be chosen in a varied manner by a person skilled in the art in order to prepare a composition having the desired properties, for example in terms of consistency or texture.

According to embodiments of the invention, the cosmetic composition comprises at least one cosmetic adjuvant chosen from cosmetically active agents, preserving agents, antioxidants, fragrances, fillers, UV-screening agents, pigments, odor absorbers and dyestuffs.

Typical active agents are moisturizers; free-radical scavengers; keratolytic and desquamating agents; vitamins; anti-elastase and anti-collagenase agents; trace elements; algal or plankton extracts; enzymes and coenzymes; flavonoids and isoflavonoids; ceramides; anti-glycation agents; NO- synthase inhibitors; agents for stimulating the synthesis of dermal or epidermal macromolecules and/or for preventing their degradation; agents for stimulating fibroblast or keratinocyte proliferation and/or keratinocyte differentiation; tensioning agents; anti-pollution agents and/or free-radical scavengers; muscle relaxants or dermo-decontracting agents, and mixtures thereof.

More in particular, examples of active agents that may be mentioned include (N-2-hydroxyethylpiperazine-N-2-ethane) sulfonic acid (HEPES); hyaluronic acid; lanolin; urea which is a typical emulsion breaker, and mixtures containing urea such as NMF (Natural Moisturizing Factor), and urea derivatives such as N-(2-hydroxy- ethyl)urea (Hydrovance from the company National Starch); 2-oxothiazolidine-4-carboxylic acid (procysteine); α-hydroxy acids, especially fruit-based acids, for instance glycolic acid, which is a typical emulsion breaker, lactic acid, malic acid, citric acid, tartaric acid or mandelic acid, and derivatives and mixtures thereof; β -hydroxy acids, for instance salicylic acid and its derivatives such as 5-n-octanoylsalicylic acid or 5-n-dodecanoylsalicylic acid; α-keto acids, for instance ascorbic acid or vitamin C and its derivatives such as its salts, for instance sodium ascorbate and magnesium or sodium ascorbyl phosphate; its esters, for instance ascorbyl acetate, ascorbyl palmitate and ascorbyl propionate, its ethers, for instance ethyl ascorbic acid, or its sugars, for instance glycosyl ascorbic acid, and mixtures thereof; β-keto acids,- retinoids, for instance retinol (vitamin A) and its esters, retinal, retinoic acid and its derivatives, and also the retinoids, carotenoids such as lycopene,- ceramides; resveratrol; pseudodipeptides such as {2-[acetyl (3-trifluoromethylphenyl) amino] -3-methylbutyryl- amino}acetic acid; vitamins, for instance, besides vitamins A and C indicated above, vitamin E (tocopherol), vitamin B3 (or vitamin PP or niacinamide), vitamin B5 (panthenol in its various forms: D-panthenol, DL-panthenol), vitamin D and vitamin F (mixture of essential fatty acids), and derivatives, precursors and analogues of these vitamins; soybean extracts, in particular soybean protein hydrolysates or isoflavone-rich soybean extracts; trace elements, for instance copper, zinc, selenium, iron, magnesium or manganese; algal extracts, plankton extracts such as the plankton in aqueous dispersion (CTFA name: Vitreoscilla ferment), enzymes, coenzymes such as ubiquinone or coenzyme QIO which belongs to the family of benzoquinones containing an alkylene chain, coenzyme R, which is biotin (or vitamin H); yeast extracts, for instance the extract of S. cerevisiae, adenosine; plant extracts, for instance extracts of liquorice; calmatives, for instance bisabolol and calmative plant extracts, for instance extracts of rose (Rosa gallica) and extracts of mint (Mentha piperita); and any active agent that is suitable for the final aim of the composition, and mixtures thereof.

The ingredients and/or active agents may range from 0.01% to 20% by weight, 0.05% to 10% or from 0.1% to 1% by weight relative to the total weight of the composition.

According to embodiments of the invention, the cosmetic composition comprises at least one UV-screening agent, preferably at least one lipophilic or liposoluble UV-screening agent. Examples of lipophilic UV-screening agents include anthranilates; cinnamic derivatives; dibenzoylmethane derivatives; salicylic derivatives; camphor derivatives; triazine derivatives; benzophenone derivatives; β,β-diphenyl-acrylate derivatives; benzotriazole derivatives; benzalmalonate derivatives, benzimidazole derivatives; imidazolines; p-aminobenzoic acid (PABA) derivatives; benzoxazole derivatives; screening polymers; α-alkylstyrene-based dimers; 4,4-diarylbutadienes and mixtures thereof. Lipophilic UV-screening agents are typically present in concentrations ranging from 0.1% to 30% by weight or 0.5% to 15% by weight relative to the total weight of the composition.

According to embodiments of the invention, the cosmetic composition comprises at least one filler. Examples of fillers include powders of natural organic materials such as corn, wheat or rice starch; or alternatively materials of natural mineral origin, for instance silica, talc, clays, for instance kaolin, montmorillonite, saponites, laponites and illites. Fillers may be present at less than or equal to 20% of the total weight of the composition, less than or equal to 10% of the total weight of the composition, less than or equal to 8%, or less than or equal to 5% of the total weight of the composition. When they are present, these fillers may be present in amounts ranging, for example, from 0.05% to 8% by weight or from 0.1% to 5% by weight relative to the total weight of the composition.

The cosmetic compositions described herein find their application in a large number of cosmetic treatments, especially cosmetic treatments for the skin, including the scalp, the hair, and/or mucous membranes, in particular for caring for, cleansing and/or sun-protecting the skin and/or mucous membranes.

According to embodiments of the invention, the cosmetic composition further comprises hydrophobic particles such as pigments that can be coated with silicones such as triethoxycaprylylsilane, dimethicone, dimethicone copolyol, perfluorooctyl triethoxysilane, trimethylsiloxysilicate, esters such as isopropyl titanium triisostearate, sodium glycerophosphate, magnesium myristate, polyperfluoromethylisopropyl ether, hydrogenated lecithin, lauroyl lysine, blends based on natural waxes, such as carnauba wax and polyethylene wax, and or vegetable oils and esters such as jojoba ester, and mixtures thereof.

Said hydrophobic particles may serve as cosmetic preservation products.

In recent years cosmetic, toiletry and pharmaceutical manufacturers have been severely limited in their choice of preservative agents. One class of biocides that has been highly effective in cosmetic, toiletry and pharmaceutical products includes formaldehyde donors, such as imidazolidinyl urea, diazolidinyl urea, and DMDM hydantoin. However, many such compounds are considered to be skin irritants and the use of formaldehyde donors is severely restricted by regulations in the EU and Japan.

Another class of preservatives includes the isothiazolinones. Methylchloroisothiazolinone is a chloro-substituted isothiazolinone that has demonstrated irritation potential and it is prohibited from use in leave-on products in some countries.

Another class of preservatives is chlorinated aromatic compounds, such as chlorphenesin. They are not broadly used in cosmetic, toiletries or pharmaceuticals because they exhibit a very strong and unpleasant odor. Also chlorinated compounds in general are used in herbicides and pesticides, and many are known human toxins, and thus chlorinated compounds may have a negative consumer perception.

Yet another class of preservatives is para-hydroxybenzoic acids, known as parabens. Preservative blends containing parabens, are the most widely used preservative systems and have been used safely and effectively for over 20 years. However, some research has suggested that parabens are possible human carcinogens. The media has suggested that products containing parabens are dangerous. Consumer groups, such as Breast Cancer Action, have lobbied cosmetic and toiletry companies to remove parabens from their products. As a result, parabens are now *de facto* banned from many segments of the cosmetic and toiletry industry.

Alcohols, and in particular diols, more specifically vicinal diols are attractive alternatives for the above preservatives. "Vicinal diols", as used herein, are materials that have hydroxyl groups which are bonded to atoms in the molecule which are next to each other, i.e., wherein two atoms each bearing a hydroxyl group are bonded to each other. Examples of vicinal diol compounds include, but are not limited to, ethylene glycol and propylene glycol. Such materials are known for use as humectants and solvents in cosmetic, toiletry and pharmaceutical products. They are also known to have some modest antimicrobial activity as described in U.S. patent application publication no. US 2007/0207105 A1, the disclosure of which is incorporated herein by reference to the extent that pertains to vicinal diols and compositions incorporating these compounds.

The most preferred vicinal diols for use in the compositions described herein when used in cosmetic, toiletry and pharmaceutical applications are medium-chain length, linear vicinal diols that demonstrate antimicrobial activity at relatively low use-levels. Such diols include 1,2-pentanediol, 1,2-hexanediol, caprylyl glycol, and 1,2-decanediol. Other vicinal diols useful in the compositions described herein include molecules derived from glycerin. Glycerin can be reacted with other molecules at its 1- or 3-position, leaving two vicinal hydroxyl groups. For example, glyceryl monoethers, such as ethylhexylglycerin [3-(2-ethylhexyloxy)propane-1,2-diol], are useful liquid vicinal diols having antimicrobial properties. Glyceryl monoesters such as glyceryl monolaurate, glyceryl mono caproate, or glyceryl monocaprylate, are also useful antimicrobial vicinal diols. For the preservation of cosmetics, toiletries and pharmaceuticals, vicinal diols are known to be effective against bacteria and yeast but weak against fungi. However, above vicinal diols, such as caprylyl glycol and glyceryl monocaprylate are typical emulsion breakers.

In general, preservatives that can be present in cosmetic formulations are listed in EC1223/2009 Annex V.

According to embodiments of the present invention, the cosmetic composition further comprises hydrophobic particles and/or alcohols such as vicinal diols and are also substantially free of parabens.

Other organic acids are anisic acid and its salts, levulenic acid and its salts, dehydroacetic acid and its salts.

It is known that the presence of molecules with electrolyte properties, e.g. salts or other molecules which have a positive or negative charge, dependent on the solubility and the pH, may induce coalescence of an emulsion, because of the dehydrating effect of the surface-active agents.

Electrolytes are a thus considered to be emulsion breakers. On the other hand, the presence of charged molecules can be desired because of its advantages for the final formulation.

According to embodiments of the present invention, the cosmetic composition further comprises at least one electrolyte.

One can use a mixture of salts, including natural mixtures or mixtures whose composition is close to that of a natural mixture, especially an aqueous mixture comprising 3 to 30 wt% magnesium chloride, 0.2-10 wt% magnesium sulfate, from 2 to 28 wt% of potassium chloride, 3 to 30 wt% sodium chloride, 0.2 to 10 wt% of calcium chloride, 0.1 to 6 wt% magnesium bromide and 0.1 to 0.5 wt% insolubles, relative to the total weight of all components, said mixture being referred to herein as "the mixture of Dead Sea salts" (Dead Sea bath salts) as it corresponds to the main salts contained in the Dead Sea.

The salts may also be in the form of a solution, in particular as a thermal or mineral water. In general, a mineral water is suitable for consumption, which is not always the case with a thermal water. Each of these waters contains, *inter alia,* dissolved minerals and trace elements.

Electrolytes may also be chosen from the active salts, for example salts derived from vitamins such as vitamin C (ascorbic acid) or acid active salts as acid salts filters. Salts derived from vitamins include e.g. ascorbyl phosphate of an alkali metal, alkaline earth or transition as magnesium, sodium, potassium, calcium, zinc; retinyl phosphate of an alkali or alkaline earth metal such as magnesium, potassium.

Salts derived filters, comprise benzenesulfonic acid salts of 1, 4-[di (3-methylidene-10-sulphonic acid)], the sodium sulphonate of 2-hydroxy-4-methoxy benzophenone-5 (or benzophenone-5), the disodium salt of 2,2'-dihydroxy-4,4'-dimethoxy-5,5'-disulfo-benzophenone (benzophenone or 9). To balance skin feel and functionality, many formulators use PBSA, 2-phenylbenzimidazole-5-sulfonic acid, a water-soluble sunscreen with excellent absorption in the UV-B wavelength region, in combination with oil-soluble UV filters. However, PBSA must be neutralized to pH 7-8 prior to use or it may revert to its acid form and crystallize, rendering it less effective as a sunscreen active. At pH 7-8, it is available in its salt form, which will negatively interact with ionic polymers, resulting in low viscosity.

In certain embodiments of the present invention, the compositions may comprise a thickener, for instance to reduce or prevent creaming. Creaming refers herein to a phase separation, typically caused by oil droplets moving towards the top of the system, but which does not implicate coalescence.

Many thickeners are polysaccharides of natural origin or plant origin. "Polysaccharides of plant origin" especially refers to polysaccharides obtained from the plant kingdom (plants or algae), as opposed to polysaccharides obtained via biotechnology, as is the case, for example, for xanthan gum, which is produced especially by fermentation of a bacterium, *Xanthomonas campestris.*

A plant-derived polysaccharide may, where appropriate, be chemically modified to promote its hydrophilic valency, as is the case for cellulose derivatives, in particular hydroxyalkyl celluloses (e.g.: hydroxyethylcellulose).

Suitable examples of polysaccharides of plant origin include algal extracts, such as alginates, carrageenans and agars, and mixtures thereof, gums, such as guar gum and nonionic derivatives thereof (hydroxypropyl guar), gum arabic, konjac gum or mannan gum, gum tragacanth, ghatti gum, karaya gum or locust bean gum, modified or unmodified starches, such as those obtained, for example, from cereals, for instance wheat, corn or rice, from legumes, for instance blonde pea, from tubers, for instance potato or cassava, and tapioca starches; dextrins, such as corn dextrins, celluloses and derivatives thereof, in particular alkyl celluloses, hydroxyalkyl celluloses; and alkyl hydroxyalkyl celluloses; mention may be made especially of methyl-celluloses, hydroxyethylcelluloses, ethyl-hydroxyethylcelluloses and carboxymethyl-celluloses. Examples that may be mentioned include stearyl and cetyl hydroxyethylcellulose.

Other suitable examples of polysaccharides are linear polyglucoses obtained by fermentation of *Sclerotium sp.,* or *Rhizobium sp.,* xanthan gum, and xanthan gum modified with glucose/mannose/glucuronic acid groups.

Thickening agents may also be synthetic. Such synthetic thickeners are typically based on polyacrylates although polyacrylamides can be equally used, such as acrylate copolymers and/or acrylate-alkyl acrylate copolymers. Also suitable are copolymers of C10.30-alkyl acrylates and one or more monomers of acrylic acid, of methacrylic acid or esters thereof which are crosslinked with an alkyl ether of sucrose or an alkyl ether of pentaerythritol. Other examples are compounds with INCI name "acrylates/C10.30 alkyl acrylate crosspolymer", Compounds with INCI name "acrylates/c12-24 pareth-25 acrylate copolymer", INCI name "acrylates/steareth-20 methacrylate copolymer", INCI name "acrylates/steareth-20 itaconate copolymer", INCI name "acrylates/aminoacrylates/C10.30 alkyl PEG-20 itaconate copolymer", and similar polymers

Mineral thickeners can be equally used. These are naturally occurring, mined ingredients that can absorb water or oils and boost viscosity. They give a different kind of viscosity than the natural gums. Materials include silica, bentonite, and magnesium aluminum silicate. These thickeners can be used to thicken oils as well as water-based formulations.

According to embodiments of the invention, the hydrophilic phase further comprises a thickening agent to prevent creaming, which thickening agent may be a thickening agent of plant origin, a synthetic agent or a mineral thickener.

An aspect according to the invention relates to the use of a cosmetic composition comprising a combination of at least one saponin as described herein and at least one prebiotic component selected from the group consisting of fructooligosaccharides, fructans, galactooligosaccharides, galactans, glucooligosaccharides, glucans, pectin, xylooligosaccharides, xylans, and mixtures thereof, in the preparation of a rinse-off cosmetic product or a leave-on cosmetic product.

According to embodiments of the invention, the rinse-off cosmetic product may be a rinse-off skin care product or a rinse-off hair care product, including hair conditioner, shampoo, shower gel, liquid soap gel, shaving cream, and the like.

According to embodiments of the invention, the leave-on cosmetic product may be a leave-on skin care product or a leave-on hair care product, including a styling hair gel, leave-on conditioner, facial cream, body lotion, lotions, a sun blocker cream, and the like.

The resulting rinse-off cosmetic products or leave-on cosmetic products are also aspects of the present invention. The use of the cosmetic composition described herein as a rinse-off cosmetic product or a leave-on cosmetic product is also an aspect of the invention.

An aspect of the present invention relates to a medicinal composition for the treatment of acne vulgaris, the medical composition comprising: at least one saponin; and at least one prebiotic component selected from the group consisting of fructo-oligosaccharides, fructans, galactooligosaccharides, galactans, glucooligosaccharides, glucans, pectin, xylooligosaccharides, xylans, and mixtures thereof. The medicinal composition may be used in a rinse-off product, such as shower gel or a facial wash. The medicinal composition may comprise the at least one saponin and the at least one prebiotic component in concentration ranges as described above for the cosmetic compositions according to the invention, in particular as described for rinse-off products. The medicinal composition may further comprise any additional components as described above for the cosmetic compositions according to the invention, in particular as described for rinse-off products.

### EXAMPLES: Effect on skin microbiota

Referring now to **Example 1,** the effect of contacting the skin with a variety of different compositions is measured for the overall skin microbiota. Following the general procedure described herein, the influence of contacting the skin with a prebiotic only, with a saponin only, and with both a saponin and a prebiotic was measured. A Shannon Diversity Index was then calculated for the variety encountered in the skin flora. The results are given in Table 1.

It is observed that even applying a "blank" solution (CE1), being water, disturbs the skin flora. This is because every change in environment on the skin will have an influence on the skin flora. The disturbance is expressed by the Shannon Diversity Index which deviates from the originally calculated value. It is furthermore observed that the application of a tea saponin solution or a soapnut saponin solution disturbs the skin flora, whereby the effect of tea saponin is much more pronounced. It is furthermore observed that an inulin solution (CE2-CE4) disturbs the skin flora even less than water, as exemplified by the deviations for the calculated Shannon diversity Index. A high deviation for the Shannon Index is found for a composition having an inulin and GMS SE (CE5), which often acts as an emulsifier in cosmetic products.

Unexpectedly, the application on the skin of a combination of inulin and at least one saponin (E1), according to the invention, resulted in small deviations which, on average, did not affect the Shannon Diversity Index, which is indicative of a synergistic effect between saponin and inulin as used in combination in the present invention.

Referring now to **Example 2,** the effect of contacting the skin with the compositions of Example 1 is measured for a variety of bacterial genera. The genera *Cutibacterium, Corinebacterium, Micrococcus, Streptococcus, Staphylococcus,* and *Kocuria* are considered to be resident and protective bacteria. The unclassified Betaproteobacteria are considered to be resident skin bacteria. The results are given in Table 2.

It is observed that even when applying the blank solution (CE6), four of the first six genera are subject to heavy changes in population. For compositions with tea saponin (CE7) or a standard emulsifier with inulin (CE10), none of the first six genera is practically left undisturbed.

It was unexpectedly found that combination of inulin and at least one saponin (E2), according to the invention, on average did not substantially affect the populations of the measured genera, with only two of the first six genera showing deviations of slightly above 30%.

Referring now to **Example 3,** the effect of contacting the skin with the compositions of Example 1 is measured for a variety of prebiotic components. The Shannon Diversity Index was calculated to measure the effect of combinations of two saponins with a different prebiotic component. The results are given in Table 3.

It is observed that for different prebiotic components, the deviation for the calculated Shannon Diversity Indices is always lower than for the case where the blank solution is applied, indicating that the effect is not limited to a single prebiotic component.

Referring to **Example 10,** it is observed that a typical rinse-off composition applied to the skin results in an increase of abundance of *Cutibacterium acnes* (formerly known as *Propionibacterium acnes*). When a part of the anionic surfactant is replaced by the same amount of a saponin together with addition of inulin, the abundance of *Cutibacterium acnes* is decreasing, showing that the disturbance of the surfactant is normalized by the combination of the saponin and prebiotic component.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

### Saponin Extraction

Another aspect according to the invention relates to a process for preparing a cosmetic composition according to any of the preceding claims, the process comprising: extracting at least one saponin from its source using a mixture of water and a water-soluble alcohol; and combining said at least one saponin with at least one prebiotic component selected from the group consisting of fructooligosaccharides, fructans, galactooligosaccharides, galactans, glucooligosaccharides, glucans, pectin, xylooligosaccharides, xylans, and mixtures thereof. Aspects of the saponin extraction step will now be described in more detail. It should be noted that while the description is presented here as part of the process for preparing a cosmetic composition, these aspects form a novel and inventive method regardless of the subsequent use of the extracted saponin in the cosmetic or medicinal compositions according to the invention.

The method pertains to extracting said at least one saponin from its plant source, by use of a mixture of water and a water-soluble alcohol.

It is known that saponins can be isolated from their plant source by extracting the saponin with water, usually demineralized water, a water-soluble alcohol or lower alcohol, or a combination of (demineralized) water and said alcohol. United States patent no. US 9,474,283, paragraphs [0132]-[0137] of which are incorporated herein by reference, describes a method for extracting saponin employing aqueous alcohol.

The presently disclosed method may comprise reducing the color intensity of a saponin extracted from a saponin source.

Typically, manufacturers of cosmetic products prefer as many of their basic ingredients to be as colorless as possible. However, most of the saponin sources or products which can be found on the market have a dark (e.g. brown) color. Even though only small amounts of saponin from these sources may be needed in a cosmetic composition, such small amounts may have a noticeable effect on the color of the final cosmetic product. It is therefore of importance to isolate the saponin from its source while reducing the color intensity, or color saturation, of the isolated product as much as possible.

Embodiments of the presently disclosed method comprise reducing the color intensity of a saponin extracted from a saponin source by said use of a mixture of water and a water-soluble alcohol, by using an alcohol:water mixture having a ratio of between 50:50 and 90:10, preferably between 55:45 and 85:15, more preferably between 60:40 and 80:20.

Embodiments of the presently disclosed method comprise reducing the color intensity of a saponin extracted from a saponin source by said use of a mixture of water and a water-soluble alcohol, by using of a combination of water and a lower alcohol for reducing the color intensity or color saturation of the saponin in the extraction process.

For the purpose of the invention, the term "lower alcohol" refers herein to a water-soluble alcohol, more in particular to an alcohol having no more than 7 carbon atoms. Preferably, said water-soluble alcohol is methanol, ethanol, 1-propanol, isopropyl alcohol or a butanol isomer.

It was now unexpectedly found that the color intensity of the extracted solution, which contains the major part of the saponin, can depend on the composition of the alcohol:water mixture. As such, a careful selection of a combination of water, preferably demineralized water, and a water-soluble alcohol, preferably methanol, ethanol, 1-propanol, isopropanol and mixtures thereof, allows reducing the color intensity, or color saturation, of the extract solution.

**Tables 11-12** show examples of extractions of saponin performed with alcohol:water mixtures with a different concentration. For each table, which show mixtures in a sequence with increasing alcohol concentration, a turning point can be found where the color intensity of the extract drops. Advantageously, the extraction yield is however not affected. It was found that starting from a certain alcohol: water ratio, a 10% increase in alcohol concentration resulted in at least a 10% drop in measured color intensity, often a 20%, 30% or even 40% reduction in color intensity.

It was thereby found that such an acceptable tradeoff between the decrease in color intensity and the extraction yield was often realized for an alcohol: water ratio which was situated between 60:40 and 80:20.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

### EXPERIMENTAL SECTION

### General procedure for assessing the invention

Compositions as described in Examples 1-4 were prepared by mixing the ingredients with the appropriate amount of demineralized water. Hence, the composition "Tea saponin 0.2%" refers to a composition that consists of 0.2 wt% tea saponin, supplemented with 99.8 wt% demineralized water, whereas the composition "blank" refers to the neutralizing diluent which is water.

As prebiotics are used:
- INUTEC H25P: fructo-oligosaccharide from chicory (CreaChem)
- INUTEC HSI: fructo-oligosaccharide from chicory (CreaChem)
- INUTEC N10: native inulin from chicory (CreaChem)
- Orafti Bio: inulin from Agave (BENEO Orafti)
- GOS: gluco-oligosaccharide (Baoling Bao Biology)

The general procedure for measuring the effect of a composition on the diversity of skin microbacteria uses the detection and enumeration of bacteria in swabs. It is known that such a semi-quantitative approach enables enumeration of the micro-organisms per cm². The procedure consisted of the following:
- A rectangular surface of 4 cm² was delimited on the skin of the inside of a lower arm for each of the volunteers.
- At time t₀, a first sterile swab was submerged in a neutralizing diluent applied over said delimited surface, thereby collecting a community of microorganisms or skin flora of said surface, and conserved in otherwise sterile conditions.
- Immediately after swabbing said delimited area with said first sterile swab, a composition as described herein was applied to said delimited area by spreading said composition over said delimited area and, depending on the test conditions, either allowing it to dry for a period of time or washing it off. For the blank experiment, no extra composition other than water was thus applied.
- For a period of one hour, starting from the application of said composition, the delimited area was kept exposed to ambient air and did not touch any other surface or liquid.
- At time t₁ = t₀ + 1 h, a second sterile swab was applied over said delimited surface, thereby collecting skin flora of said surface, and was in its turn conserved in other sterile conditions.

The swabs thus obtained were all examined within 24 hours of collection. Prior to examination, said swabs were kept at a temperature of 2-8 °C. The swabs were vortex mixed or homogenized to aid release of organisms into the diluent.

Isolated DNA was then amplified by PCR amplification using primer pairs 347F and 803R (V3-V4) covering hypervariable regions of the 16S rRNA gene. It is known that 16S sequencing allows for the identification of bacterial species present in a community and to separate similar strains. The resulting libraries were sequenced using the Illumina MiSeq tool. Data sets were obtained in FASTQ format were denoised and analyzed.

Analysis of the obtained data sets allowed determining the numbers of bacteria of specific genera of skin flora that had been picked up by the swabs. These numbers provide evidence of any decrease or an increase in the prevalence of any specific genus of bacteria due to the application of the composition under test to the skin.

A Shannon Diversity Index could be calculated, as indicated here above.

### Example 1 - Measuring to what degree applying different types of compositions disturbs the overall diversity of skin microbiota

The effects on the overall composition of skin microbiota by applying a composition to the skin according to the general procedure described here above were measured for six different compositions. The contact time of the compositions was 1 h. The compositions were not rinsed off. The results are given in Table 1. The Shannon Diversity Indices for the overall community of microorganisms were calculated according to the formula as described herein.

**Table 1**

| | | Blank (CE 1) | | Tea saponin 0,2% (CE2) | | Soapnut saponin 0,2% (CE 3) | | INUTEC N10 1% (CE 4) | | Tea saponin 0,1%+Soapnut saponin 0,1% + INUTEC N10 1% (E 1) | | GMS SE 5% + Inulin 1% (CE 5) | |
|---|---|---|---|---|---|---|---|---|---|---|---|---|---|
| Volunteer 1 | 0h | 3,8 | 5,3% | 4,2 | 7,1% | 4,1 | 9,8% | 3,9 | 0,0% | 4,9 | 2,0% | 3,7 | 8,1% |
| | 1h | 4 | | 3,9 | | 4,5 | | 3,9 | | 4,8 | | 4 | |
| Volunteer 2 | 0h | 7,2 | 6,9% | 3,8 | 13,2% | 4,7 | 12,8% | 7,6 | 7,9% | 5,5 | 3,6% | 7,3 | 9,6% |
| | 1h | 7,7 | | 4,3 | | 5,3 | | 8,2 | | 5,3 | | 8 | |
| Volunteer 3 | 0h | 4,2 | 7,1% | 4,1 | 68,3% | 4,2 | 2,4% | 4,1 | 4,9% | 4,2 | 4,8% | 4,3 | 14,0% |
| | 1h | 4,5 | | 1,3 | | 4,3 | | 4,3 | | 4,4 | | 4,9 | |
| Average | | 0,3 | 6,5% | 0,9 | 29,5% | 0,4 | 8,3% | 0,3 | 4,3% | 0 | 3,5% | 0,5 | 10,6% |

### Example 2 - Measuring to what degree applying different types of compositions disturbs the diversity of skin microbiota - per genus

The effects on a set of bacterial genera of applying a composition to the skin according to the general procedure described hereinabove were measured for six different compositions. The contact time was 1 h. The compositions were not rinsed off. The results are given in Table 2. For each of said six compositions, the table shows per genus, how the overall quantity of the bacterial population changed on a percentage basis. It is thereby considered that a deviation higher than 30% illustrates a significant influence of the applied composition.

**Table 2**

| Deviation in % | Blank (CE6) | Tea saponin 60% (Choisun) 0.2% (CE7) | Soapnut saponin 70% (Naturalin) 0.2% (CE8) | INUTEC N10 1% (CE9) | Tea saponin 60% (Choisun) 0,1%+ Soapnut saponin 70% (Naturalin) 0,1% + INUTEC N10 1% (E2) | GMS SE 5% + INUTEC N101% (CE10) |
|---|---|---|---|---|---|---|
| Cutibacterium | 17 | 70 | 23 | 29 | 17 | 35 |
| Corynebacterium | 31 | 35 | 54 | 37 | 37 | 43 |
| Streptococcus | 39 | 54 | 20 | 28 | 20 | 40 |
| Staphylococcus | 37 | 86 | 108 | 41 | 34 | 51 |
| Kocuria | 27 | 33 | 27 | 37 | 25 | 50 |
| Micrococcus | 32 | 41 | 32 | 32 | 18 | 36 |
| Unclassified Beta proteobacteria | 100 | 83 | 76 | 113 | 60 | 380 |

### Example 3 - Measuring to what degree applying different types of compositions disturbs the diversity of skin microbiota - variation of the prebiotic component

The effects on the overall composition of skin microbiota by applying a composition to the skin according to the general procedure described here above were measured for six different compositions. Contact time on the skin was 60 seconds, followed by a rinse-off with demineralized water. The results are given in Table 3. The Shannon Diversity Indices for the overall community of microorganisms were calculated according to the formula as described herein.

**Table 3**

| | | Blank (CE11) | Tea saponin 60% (Choisun) 0,01% + Soapnut saponin 70% (Naturalin) 0,19% + INUTEC N10 1% (E3) | Tea saponin 60% (Choisun) 0,01% + Soapnut saponin 70% (Naturalin) 0,19% 0,19% + GOS 1% (E4) | Tea saponin 60% (Choisun) 0,01% + Soapnut saponin 70% (Naturalin) 0,19% + Orafti Bio 1% (E5) |
|---|---|---|---|---|---|
| Volunteer 1 | 0h | 3.8 | 4.2 | 3.7 | 3.6 |
| | 1h | 4 | 3.9 | 3.4 | 3.7 |
| Volunteer 2 | 0h | 7.2 | 7.2 | 7 | 6.9 |
| | 1h | 7.7 | 7.1 | 7.1 | 6.3 |
| Volunteer 3 | 0h | 4.2 | 4.1 | 4.3 | 4 |
| | 1h | 4.5 | 4.3 | 4.8 | 4.2 |
| Average | | 0.3 | 0 | 0.1 | -0.1 |

### Example 4 - Emulsions of cosmetic compositions - increasing presence of oil

**Table 4**

| wt% | E6 | E7 | E8 | E9 | E10 | E11 | E12 | E13 |
|---|---|---|---|---|---|---|---|---|
| H₂O | qs. | qs. | qs. | qs. | qs. | qs. | qs. | qs |
| Orafti Bio | 1 | 1 | 1 | 1 | 1 | 1 | 1 | 1 |
| Xanthan gum | 0.6 | 0.6 | 0.6 | 0.6 | 0.6 | 0.6 | 0.6 | 0.6 |
| Ethylhexylglycerin, Phenoxyethanol | 1 | 1 | 1 | 1 | 1 | 1 | 1 | 1 |
| Glycerin | 3 | 3 | 3 | 3 | 3 | 3 | 3 | 3 |
| Tea Saponin 60% (Choisun) | 0.5 | 0.5 | 0.5 | 0.5 | 0.5 | 0.5 | 0.5 | 0.5 |
| Cetearyl Alcohol C1618 50/50 | 2 | 2 | 2 | 2 | 2 | 2 | 2 | 2 |
| Caprylic/Capric Triglyceride | 10 | 20 | 30 | 40 | 50 | 60 | 70 | 90 |
| NaOH 10% pH 6-7 | qs. | qs. | qs. | qs. | qs. | qs. | qs. | qs |

According to the general principles described hereabove, emulsions of cosmetic compositions E6-E12 according to the invention were prepared. The emulsions are illustrated in Table 4. All prepared emulsions were stable against coalescence after 4 weeks storage at 50°C.

### Example 5 - Emulsions of cosmetic compositions - different types of oil

According to the general principles described hereabove, cosmetic compositions E13-1 to E13-44 according to the invention were prepared, wherein the parameter "oil" refers to one of the following:
(1) Macadamia oil; (2) Jojoba oil; (3) Sweet Almond oil; (4) Soybean oil; (5) Ethylhexyl Stearate; (6) Decyl Oleate; (7) Isoamyl laurate; (8) Cetearyl Ethylhexanoate; (9) Isodecyl Neopenanoate; (10) Octyldodecanol; (11) Cetyl palmitate; (12) Isostearyl Isostearate; (13) Isononyl Isononanoate; (14) C12-15 Alkyl Benzoate; (15) Isopropyl Palmitate; (16) Isopropyl Mirystate; (17) Cetearyl Isononanoate; (18) Pentaerythrityl Tetracaprylate/Tetracaprate; (19) Ethylhexyl Palmitate; (20) PEG-6 Caprylic/Capric glycerides; (21) Coco-Caprylate; (22) Triethyl Citrate; (23) Peg-7 Glyceryl Cocoate; (24) Dicaprylyl Ether; (25) Coco-Capryalte/Caprate; (26) Dimethicone M100; (27) Cyclopentasiloxane; (28) Cyclopentasiloxane, Dimethicone; (29) Cyclopentasiloxane, Dimethiconol; (30) Dimethicone, Cetearyl Dimethicone Crosspolymer; (31) Cyclopentasiloxane, Cyclohexasiloxane; (32) Cyclopentasiloxane, C30-45 Alkyl Cetearyl Dimethicone Crosspolymer; (33) Aqua, Amodimethicone, C11-15 Pareth-7, Laureth-9, Glycerin, Trideceth-12; (34) Mineral oil; (35) Polydecene; (36) Isododecane; (37) Isohexadecane; (38) Squalane; (39) Alkyl Benzoate, Polysilicone-15; (40) Alkyl Benzoate, Ethylhexyl Methoxycinnamate; (41) Alkyl Benzoate, Butyl Methoxydibenzoylmethane; (42) Octocrylene; (43) Benzophenone-3; (44) Ethylhexyl triazone. The emulsions are illustrated in Table 5. All compositions were stable against coalescence after 4 weeks storage at 50 °C.

**Table 5**

| | E13-(1-44) |
|---|---|
| H₂O | qs. |
| Inulin (INUTEC N10) | 1 |
| xanthan gum | 0.6 |
| Ethylhexylglycerin, Phenoxyethanol | 1 |
| Glycerin | 3 |
| Tea saponin 60% (Choisun) | 0.5 |
| Cetearyl Alcohol C1618 50/50 | 2 |
| Oil (1 to 44) | 20 |
| NaOH 10% pH:6-7 | qs. |

### Example 6 - emulsions of cosmetic compositions - different types of saponin

**Table 6**

| (wt%) | E14 | E15 | E16 | E17 |
|---|---|---|---|---|
| H₂O | qs. | qs. | qs. | qs. |
| Inulin (INUTEC HSI) | 1.5 | 1.5 | 1.5 | 1.5 |
| xanthan gum | *0.6* | 0.6 | 0.6 | 0.6 |
| Ethylhexylglycerin, Phenoxyethanol | 1 | 1 | 1 | 1 |
| Glycerin | 3 | 3 | 3 | 3 |
| Cetearyl Alcohol C1618 50/50 | 2 | 2 | 2 | 2 |
| Caprylic/Capric Triglyceride | 6*0* | 6*0* | 6*0* | 6*0* |
| NaoOH 10% pH:6-7 | qs. | qs. | qs. | qs. |
| Quillaja Saponaria 67% (Desert King) | 0.5 | - | - | - |
| Gynostemma pentaphyllum 60% (Greenherb) | - | 0.5 | - | - |
| Soapnut 70% (Naturalin) | - | - | 0.5 | - |
| Green tea (50%) (Vital-Chem) | - | - | - | 0.5 |

According to the general principles described hereabove, emulsions of cosmetic compositions E14-E17 according to the invention were prepared. The emulsions are illustrated in Table 6. All prepared emulsions were stable against coalescence after 4 weeks storage at 50°C.

### Example 7 - emulsions of cosmetic compositions - different types of emulsion breakers

**Table 7**

| (wt%) | E18 | E19 | E20 | E21 | E22 | E23 | E24 | E25 | E26 | E27 | E28 | E29 |
|---|---|---|---|---|---|---|---|---|---|---|---|---|
| H2O | qs. | qs. | qs. | qs. | qs. | qs. | qs. | qs. | qs. | qs. | qs. | qs. |
| Inulin (INUTEC N10) | 0.5 | 0.5 | 0.5 | 0.5 | 0.5 | 0.5 | 0.5 | 0.5 | 0.5 | 0.5 | 0.5 | 0.5 |
| Hydroxyethyl cellulose | | | | | | | | 0.8 | | | | |
| Xanthan gum | 0.6 | 0.6 | 0.6 | 0.6 | 0.6 | 0.6 | 0.6 | | 0.6 | 0.6 | 0.6 | 0.6 |
| Ethylhexylglycerin, Phenoxyethanol | 1 | 1 | 1 | 1 | 1 | 1 | 1 | 1 | 1 | 1 | 1 | 1 |
| Glycerin | 3 | 3 | 3 | 3 | 3 | 3 | 3 | 3 | 3 | 3 | 3 | 3 |
| Tea saponin 90% (Plantnat) | 0.5 | 0.5 | 0.5 | 0.5 | 0.5 | 0.5 | 0.5 | 0.5 | 0.5 | 0.5 | 0.5 | 0.5 |
| Cetearyl alcohol | 2 | 2 | 2 | 2 | 2 | 2 | 2 | 2 | 2 | 2 | 2 | 2 |
| Caprylic/Capric Triglyceride | 20 | 20 | 20 | 20 | 20 | 20 | 20 | 20 | 20 | 20 | 20 | 20 |
| MgSO4 | 5 | - | - | - | - | - | - | - | - | - | - | - |
| NaCl | - | 5 | - | - | - | - | - | - | - | - | - | - |
| Dihydroxy acetone | - | - | 4 | - | - | - | - | - | - | - | - | - |
| Potassium Thioglycolate | - | - | - | 5 | - | - | - | - | - | - | - | - |
| Glycolic acid 70% | - | - | - | - | 10 | - | - | - | - | - | - | - |
| Glyceryl caprylate | - | - | - | - | - | 1.5 | - | - | - | - | - | - |
| Caprylyl glycol | - | - | - | - | - | - | 1.5 | - | - | - | - | - |
| Cetrimonium chloride CTA25 | - | - | - | - | - | - | - | 4 | - | - | - | - |
| Aqua, Cocamidopropyl Betaine | - | - | - | - | - | - | - | - | 5 | - | - | - |
| Aqua, Sodium Cocoyl Glutamate, Sodium | - | - | - | - | - | - | - | - | - | 10 | - | - |
| Hydrogen peroxide (30%) | - | - | - | - | - | - | - | - | - | - | 30 | - |
| Ethanol | - | - | - | - | - | - | - | - | - | - | - | 10 |
| pH corrected with NaOH/citric acid to | 5.3 | 5.3 | 4.3 | 10.9 | 3.5 | 6 | 6.4 | 6.5 | 6 | 6 | 6.8 | 3.6 |

According to the general principles described hereabove, emulsions of cosmetic compositions E18-E29 according to the invention were prepared. Table 7 discloses said cosmetic compositions, each containing a different emulsion breaking agent.

Unexpectedly, all prepared emulsions proved stable against coalescence after 4 weeks storage at 50 °C.

### Example 8 - emulsions of cosmetic compositions - W/O/W

**Table 8**

| (wt%) | | E30 |
|---|---|---|
| A | Aqua | 59 |
| A | Glycerin | 3 |
| A | Magnesium Sulfate | 1 |
| A | Phenoxyethanol, Ethylhexylglycerin | 0.5 |
| A | Polyglyceryl-3 Polyricinoleate, Polyglyceryl-3 Ricinoleate | 5 |
| | | |
| B | Macadamia Integrifolia Seed Oil | 10.5 |
| B | Ethylhexyl Stearate | 10.5 |
| B | Isohexadecane | 10.5 |
| | | |
| C | Aqua | 58.7 |
| C | Inulin (INUTEC H25P) | 0.5 |
| C | Glycerin | 3 |
| C | Tea saponin extract 5% + Soapnut extract 95% | 0.5 |
| C | Phenoxyethanol, Ethylhexylglycerin | 0.5 |
| C | Xanthan Gum | 0.6 |

The W/O/W emulsion, as disclosed in Table 8, were obtained by use of the following procedure: the ingredients listed under "A", "B" and "C" were mixed in three separate containers until homogeneous mixtures were obtained. The mixture with "A" ingredients was subsequently added to the "B" mixture, homogenized in a centrifuge during 5 minutes under 12000 RPM and further mixed for a period of 30 minutes.

36.2 g of the homogenised mixture was then mixed with "C" mixture at 60 °C, and further homogenized in a centrifuge for a period of 3 minutes at 10000 RPM. The W/O/W emulsion was stable for coalescence for at least 4 weeks when stored at 50 °C uninterruptedly.

### Example 9 - emulsions of cosmetic compositions - different types of surfactant

**Table 9**

| (wt%) | CE12 | E31 | CE13 | CE14 | CE15 | CE16 |
|---|---|---|---|---|---|---|
| H₂O | 82.9 | 82.9 | 82.9 | 82.9 | 82.9 | 82.9 |
| Inulin (INUTEC HSI) | - | 0.4 | - | 0.4 | - | 0.4 |
| Xanthan gum | 0.6 | 0.6 | 0.6 | 0.6 | 0.6 | 0.6 |
| Ethylhexylglycerin, Phenoxyethanol | 1 | 1 | 1 | 1 | 1 | 1 |
| Glycerine | 3 | 3 | 3 | 3 | 3 | 3 |
| Soapnut Saponin (60%) | 0.5 | - | - | - | - | - |
| | - | 0.5 | - | - | - | - |
| Steareth-21 | - | - | 0.5 | - | - | - |
| | - | - | - | 0.5 | - | - |
| Glyceryl Stearate, PEG-100 Stearate | - | - | - | - | 0.5 | - |
| | - | - | - | - | - | 0.5 |
| Cetearyl Alcohol C1618 50/50 | 2 | 2 | 2 | 2 | 2 | 2 |
| Caprylic/Capric Triglyceride | 10 | 10 | 10 | 10 | 10 | 10 |
| Citric acid pH:6-7 | qs. | qs. | qs. | qs. | qs. | qs. |

According to the general principles described hereabove, emulsions of cosmetic composition E31 according to the invention as well as comparative examples CE12 to CE16 were prepared as presented in Table 9.

The emulsions prepared with the soapnut saponin extract proved stable for coalescence for at least 4 weeks when stored at 50°C. In strong contrast therewith, the emulsions prepared with Steareth-2 and (Glyceryl Stearate, PEG-100 Stearate) as surfactants, showed coalescence after 1 week at 40 °C.

### Example 10 - Measuring to what degree substitution of an anionic surfactant by saponin + addition of inulin can normalize the presence of Cutibacterium acnes

The effects on the prevalence of *Cutibacterium acnes* on the skin by by applying a composition to the skin according to the general procedure described here above were measured for a typical rinse-off composition (CE 17) and a composition according to the invention (E 31). The components of these compositions are shown in Table 10a.

The contact time on the skin was 60 seconds, followed by a rinse-off with demineralized water. The results are given in Table 10b. It is observed that a typical rinse-off composition applied to the skin results in an increase of abundance of *Cutibacterium acnes.* When a part of the anionic surfactant is replaced by the same amount of a saponin together with addition of inulin, the abundance of *Cutibacterium acnes* is decreasing, showing that the disturbance of the surfactant is normalized by the combination of the saponin and prebiotic component.

**Table 10a**

| | CE 17 | E 31 |
|---|---|---|
| Water | qs | qs |
| Inutec N10 | | 2 |
| Soapnut saponin extract | | 0.95 |
| Tea Saponin extract | | 0.05 |
| SLES (28%), 100% | 10.7 | 9.7 |
| Cocamidopropyl Betain | 5 | 5 |
| PEG-4 Rapeseedamide (100%) | 1 | 1 |
| NaCl | 0.5 | 0.5 |

**Table 10b**

| | | CE 12 | | E 5' | |
|---|---|---|---|---|---|
| Volunteer 1 | 0h | 27.35 | 10.60% | 26.78 | -15.20% |
| | 1h | 30.25 | | 22.71 | |
| Volunteer 2 | 0h | 10.75 | 51.16% | 22.57 | -46.33% |
| | 1h | 16.25 | | 11.04 | |
| Volunteer 3 | 0h | 1.59 | 49.69% | 2.24 | -20.95% |
| | 1h | 2.38 | | 1.77 | |
| Average | | 9.19 | 37.15% | -16.07 | -27.49% |

### Example 11 - Extraction of Saponins from Sapindus Mukorossi shells by Ethanol/Water mixtures

100 grams of "as such" *Sapindus Mukorossi* (Vehgroshop) shells and 400 grams of 100% demineralized water or an ethanol/water mixture (50:50, 60:40, 70:30 and 75:25 ratios) were weighted.

The ethanol/water mixture was obtained by mixing the appropriate amounts of 96 wt% EtOH and 100% demineralized water. The demineralized water or EtOH/H2O mixture were preheated to 60 °C.

The finely ground Soapnut shells were added to the heated demineralized water or heated ethanol/water mixture and were stirred for 2 hours at 60°C using a 3-bladed propeller stirrer (950-1050 rpm). Alternatively, a magnetic stirrer (900-1080 rpm) could be used. The hot extract was filtered through a Whatman 1 filter (Qualitative 125 mm Ø ^{∗}100 circles).

After filtration, a colored solution was obtained. The EtOH in the solution was then removed via evaporation under vacuum. The color intensity of the extract after ethanol evaporation was determined according to the procedure described earlier.

Table 11 displays an overview of the color intensity measurements of the extracts and their yields.

**Table 11**

| Solvent system used | Color intensity extract | Extraction Yield |
|---|---|---|
| 100 % H₂O | 14792 | 77.23 |
| 50% EtOH:50% H₂O | 13679 | 72.31 |
| 60% EtOH:40% H₂O | 13393 | 84 |
| 70% EtOH:30% H₂O | 10283 | 78.9 |
| 75% EtOH:25% H₂O | 8857 | 77.16 |

### Example 12 - Extraction of Saponins from Sapindus Mukorossi shells by Alcohol/Water mixtures

100 grams of "as such" *Sapindus Mukorossi* (National Organic's) shells and 400 grams of 100% demineralized water, an ethanol/water mixture (50:50, 60:40, 75:25 and 85:15 ratios) or a n-butanol/water mixture (60:40, 80:20) were weighted. The ethanol/water mixture was obtained by mixing the appropriate amounts of 96 wt% EtOH and 100% demineralized water. The n-butanol/water mixture was obtained by mixing the appropriate amounts of >99.5% n-butanol and 100% demineralized water. The demineralized water or alcohol/H₂O mixtures were preheated to 60 °C. The same procedure as in Example 17 for extracting the saponins was followed.
Table 12 displays an overview of the color results of the extracts and their yields.

**Table 12**

| Solvent system used | Color intensity extract | Extraction Yield |
|---|---|---|
| 100 % H₂O | 10490 | 72.5 |
| 50% EtOH:50% H₂O | 10088 | 81.1 |
| 60% EtOH:40% H₂O | 10300 | 70.9 |
| 75% EtOH:25% H₂O | 5504 | 69.4 |
| 85% EtOH:15% H₂O | 5182 | 65.1 |
| 60% n-BuOH:40% H₂O | 10000 | 65.4 |
| 80% n-BuOH:20% H₂O | 5138 | 55,6 |

### Example 13 - Extraction of Saponins from Camellia Oleifera by Alcohol/Water mixtures

100 grams of "as such" *Camelia Oleifera* seed meal and 400 grams of 100% demineralized water, an ethanol/H2O mixture (50:50, 60:40 and 75:25 ratios) or an isopropanol/H2O mixture (60:40 and 80:20 ratios) were weighted. The ethanol/water mixture was obtained by mixing the appropriate amounts of 96 wt% EtOH and 100% demineralized water. The isopropanol/water mixture was obtained by mixing the appropriate amounts of >99.5 wt% isopropanol and 100% demineralized water. The demineralized water or alcohol/H₂O mixtures were preheated to 60°C.
The meal was added to the heated iPOH mixture and then it was stirred for 2 hours with magnetic stirring (900-1080 rpm). The hot extract was filtered through Whatman 1 (Qualitative 125 mm Ø ^{∗} 100 circles).

After filtration, a colored solution was obtained. The alcohol in the solution was then removed via evaporation under vacuum. The color intensity of the extract after ethanol evaporation was determined according to the procedure described earlier.

Table 13 displays an overview of the color intensity measurements of the extracts and their yields.

**Table 13**

| **Solvent system used** | **Color intensity extract** | **Extraction Yield** |
|---|---|---|
| 100 % H₂O | 89405 | 21.64 |
| 50% EtOH:50% H₂O | 96833 | 27.36 |
| 60% EtOH:40% H₂O | 86693 | 26.21 |
| 75% EtOH:25% H₂O | 75500 | 24.8 |
| 80% EtOH:20% H₂O | 66100 | 24.6 |
| 60% iPOH:40% H₂O | 89732 | 29.8 |
| 80% iPOH:20% H₂O | 72666 | 28.87 |

## Claims

1. A cosmetic composition comprising:
at least one saponin; and
at least one prebiotic component selected from the group consisting of fructo-oligosaccharides, fructans, galactooligosaccharides, galactans, glucooligosaccharides, glucans, pectin, xylooligosaccharides, xylans, and mixtures thereof.

2. The cosmetic composition according to claim 1, wherein said at least one prebiotic component comprises an inulin-type polymer.

3. The cosmetic composition according to any of the preceding claims, wherein said at least one prebiotic component is present in the composition in a concentration of at least 0.01 wt% to at most 8 wt%, by weight relative to the total weight of the composition.

4. The cosmetic composition according to any of the preceding claims, wherein said at least one saponin is present in the composition in a concentration of at least 0.001 wt% to at most 15 wt%, by weight relative to the total weight of the composition.

5. The cosmetic composition according to any one of the preceding claims, wherein said composition further comprises at least one of the following: an anionic surfactant, a cationic surfactant, an amphoteric surfactant, and a non-ionic surfactant, wherein said non-ionic surfactant is exclusive of a saponin.

6. The cosmetic composition according to any of the preceding claims, further comprising at least one oil selected from jojoba oil, isoamyl laurate, and cyclopentasiloxane; said at least one oil being comprised in a hydrophobic phase of a stable emulsion.

7. The cosmetic composition according to any of the preceding claims, further comprising at least one emulsion breaking agent selected from the following:
- an antimicrobial agent, such as a medium-chain length linear vicinal diol, glyceryl caprylate, or caprylyl glycol;
- an electrolyte, such as MgSO4 or NaCl;
- a cationic molecule, such as cationic surfactants such as cetrimonium chloride;
- an oxidising agent, such as hydrogen peroxide in a concentration between 10 and 30% (v/v);
- a sunless tanning agent, such as dihydroxy acetone (DHA);
- a reducing agent, such as potassium thioglycolate;
- an alpha hydroxy acid, such as glycolic acid;
- an alpha and beta hydroxy acid, such as salicylic acid;
- a surfactant, such as cocamidopropyl betaine, sodium cocoyl glutamate, or sodium lauroyl glutamate;
- a C₁-C₇ alcohol, such as ethanol.

8. The cosmetic composition according to any of the preceding claims, wherein said composition is a leave-on cosmetic product or is part of a leave-on cosmetic product, wherein said at least one prebiotic component is present in the cosmetic product in a concentration of at least 0.01 wt% to at most 8 wt%, by weight relative to the total weight of the cosmetic product.

9. The cosmetic composition according to claim 8, wherein said at least one saponin is present in the cosmetic product in a concentration of at least 0.001 wt% to at most 5 wt% by weight relative to the total weight of the cosmetic product.

10. The cosmetic composition according to claim 8 or claim 9, wherein the weight ratio of said at least one saponin to the total of all surfactants in the composition, excluding said at least one saponin, is at least 1:100 and at most 100:1.

11. The cosmetic composition according to any of the preceding claims, wherein said composition is a rinse-off cosmetic product or is part of a rinse-off cosmetic product, wherein said at least one prebiotic component is present in the cosmetic product in a proportion of at least 0.01 wt% to at most 8 wt%, by weight relative to the total weight of the cosmetic product.

12. The cosmetic composition according to claim 11, wherein said at least one saponin is present in the cosmetic product in a concentration of at least 0.1 wt% to at most 15 wt% by weight relative to the total weight of the cosmetic product.

13. The cosmetic composition according to claim 11 or claim 12, wherein the weight ratio of said at least one saponin to the total of all surfactants in the composition, excluding said at least one saponin, is at least 1:100 and at most 100:1.

14. A process for preparing a cosmetic composition according to any of the preceding claims, the process comprising:
- extracting at least one saponin from its source using a mixture of water and a watersoluble alcohol; and
- combining said at least one saponin with at least one prebiotic component selected from the group consisting of fructooligosaccharides, fructans, galactooligosaccharides, galactans, glucooligosaccharides, glucans, pectin, xylooligosaccharides, xylans, and mixtures thereof.

15. The process according to claim 14, wherein said extraction is performed by use of a mixture of ethanol and water, wherein the ethanol:water ratio is between 60:40 and 80:20.

16. Use of the cosmetic composition according to any one of claims 1-15 in the preparation of a rinse-off cosmetic product or a leave-on cosmetic product.

17. A medicinal composition for the treatment of acne vulgaris, the medical composition comprising:
at least one saponin; and
at least one prebiotic component selected from the group consisting of fructooligosaccharides, fructans, galactooligosaccharides, galactans, glucooligosaccharides, glucans, pectin, xylooligosaccharides, xylans, and mixtures thereof.
